# EUROPEAN PATENT SPECIFICATION

(11) **EP 0 564 502 B1**
(45) Date of publication and mention of the grant of the patent: **30.01.2002**
(21) Application number: 92901268.0
(22) Date of filing: 27.11.1991
(51) Int. Cl.: A61K 38/18, A61K 38/30, A61K 38/36, A61L 24/10, C12N 5/00

(54) **TISSUE SEALANT AND GROWTH FACTOR CONTAINING COMPOSITIONS THAT PROMOTE ACCELERATED WOUND HEALING**
GEWEBEABDECKUNG UND WACHSTUMSFAKTOR ENTHALTENDE VERBINDUNGEN ZUR FÖRDERUNG BESCHLEUNIGTER WUNDHEILUNG
COMPOSITIONS CONTENANT UN AGENT DE SCELLEMENT TISSUlAIRE ET DES FACTEURS DE CROISSANCE, QUI ACCELERENT LA CICATRISATION

(30) Priority: 27.11.1990 US 618419
(43) Date of publication of application: 13.10.1993
(60) Divisional application: 01113651.2
(73) Proprietor: The American National Red Cross, Falls Church, VA 22042 (US); LOYOLA UNIVERSITY OF CHICAGO, Chicago, IL 60611 (US)
(72) Inventor: GREISLER; HOWARD P., Chicago, IL 60610 (US); NUNEZ, Hernan A., Derwood, MD 20855 (US); DROHAN, William N., Springfield, VA 22152 (US); BURGESS, Wilson H., Gaithersburg, MD 20877 (US); MACIAG, Thomas, Rockville, MD 20852 (US); LASA, Jr, Carlos I., Germantown, Maryland 20874 (US)
(74) Representative: Ritter, Stephen David
(86) International application number: US9108869
(87) International publication number: WO9209301

(56) References cited:
- EP-A- 0 312 208
- WO-A-86/00526
- WO-A-86/03122
- WO-A-91/09573
- GB-A- 2 137 209
- US-A- 4 298 598
- US-A- 4 377 572
- US-A- 4 861 757
- US-A- 4 874 746
- US-A- 4 952 403
- US-A- 4 983 581
- US-A- 5 019 559
- US-A- 5 034 375
- US-A- 5 035 887
- European Journal of Clinical Investigation, Volume 18, No. 4, issued August 1988, R.R. LOBB, "Clinical Applications of Heparin-binding Growth Factors", pages 321-336, see pages 326-327.

## Description

This application is a continuation-in-part of United States Patent Application Serial No. 07/618,419 which was filed on November 27, 1990, and which is herein incorporated by reference.

### FIELD OF INVENTION

This invention is directed to growth factor-supplemented tissue sealants, such as fibrin glue, that promote: accelerated wound healing, such as of the skin and bone; endothelialization of vascular prostheses; and the *in vitro* proliferation and/or differentiation of animal cells, such as endothelial cells and fibroblasts. This invention is further directed to providing a means for delivering and maintaining prolonged contact between a growth factor(s) - supplemented fibrin glue and wounds, vascular protheses and cells growing *in vitro.*

### BACKGROUND OF THE INVENTION

Wound healing, the repair of lesions, begins almost instantly after injury. It requires the successive coordinated function of a variety of cells and the close regulation of degradative and regenerative steps. It involves fibrin clot formation, resorption of the clot, and epithelialization. Wound healing involves the formation of highly vascularized tissue that contains numerous capillaries, many active fibroblasts, and abundant collagen fibrils. but not the formation of specialized skin structures.

The process of wound healing can be initiated by thromboplastin which flows out of injured cells. Thromboplastin contacts plasma factor Vll to form factor X activator. which then, with factor V and in a complex with phospholipids and calcium, converts prothrombin into thrombin. Thrombin catalyzes the release of fibrinopeptides A and B from fibrinogen to produce fibrin monomers, which aggregate to form fibrin filaments. Thrombin also activates the transglutaminase, factor XIIIa, which catalyzes the formation of isopeptide bonds to covalently cross-link the fibrin filaments. Alpha₂-antiplasmin is then bound by factor XIII onto the fibrin filaments to thereby protect the filaments from degradation by plasmin.

When a tissue is injured, polypeptide growth factors which exhibit an array of biological activities are released into the wound where they play a crucial role in healing (see, *e.g., Hormonal Proteins and Peptides,* Li, C.H., ed., Volume 7, Academic Press, Inc. New York, pp. 231-277 and Brunt *et al*., *Biotechnology 6*:25-30 (1988)). These activities include, recruiting cells, such as leukocytes and fibroblasts, into the injured area, and inducing cell proliferation and differentiation. Growth factors that participate in wound healing include: platelet-derived growth factor (PDGF), insulin-binding growth factor-1 (IGF-1), insulin-binding growth factor-2 (IGF-2), epidermal growth factor (EGF), transforming growth factor-α (TGF-α), transforming growth factor-β (TGF-β), platelet factor 4 (PF-4), and heparin binding growth factors one and two (HBGF-1 and HBGF-2).

PDGFs, which are stored in the alpha granules of circulating platelets, are released at wound sites during blood clotting (see, *e.g.,* Lynch *et al*., *J. Clin. Invest. 84*:640-646 (1989)). PDGFs include: PDGF, platelet derived angiogenesis factor (PDAF), TGF-β and PF-4, which is a chemoattractant for neutrophils (see, Knighton *et al.,* in *Growth Factors and Other Aspects of Wound Healing: Biological and Clinical Implications,* Alan R. Liss, Inc., pp. 319-329 (1988)). PDGF is a mitogen and chemoattractant for fibroblasts and smooth muscle cells and is a stimulator of protein synthesis in cells of mesenchymal origin, including fibroblasts and smooth muscle cells. PDGF is also a nonmitogenic chemoattractant for endothelial cells.

IGF-1 acts in combination with PDGF to promote mitogenesis and protein synthesis in mesenchymal cells in culture. Application of either PDGF or IGF-1 to skin wounds alone does not enhance healing, but application of both factors together appears to promote connective tissue and epithelial tissue growth (Lynch *et al., Proc*. *Natl. Acad. Sci. 76*:1279-1283 (1987)).

TGF-β is a chemoattractant for macrophages and monocytes. Depending upon the presence or absence of other growth factors, TGF-β may stimulate or inhibit the growth of many cell types. For example, when applied *in vivo,* TGF-β increases the tensile strength of healing dermal wounds. TGF-β also inhibits endothelial cell mitosis, and stimulates collagen and glycosaminoglycan synthesis by fibroblasts.

Other growth factors, such as EGF, TGF-α, the HBGFs and osteogenin, are also important in wound healing. EGF, which is found in gastric secretions and saliva, and TGF-α, which is made by both normal and transformed cells, are structurally related and may recognize the same receptors, which mediate cell proliferation on epithelial cells. Both factors accelerate reepithelialization of skin wounds. Osteogenin, which has been purified from demineralized bone, appears to promote bone growth (see, Luyten *et al., J. Biol. Chem. 264*:13377 (1989)).

The HBGFs, which include the acidic HBGF-1 and the basic HBGF-2 polypeptides, are potent mitogens for cells of mesodermal and neuroectodermal lineages, including endothelial cells (see, *e*.*g*., Burgess *et al., Ann*. *Rev. Biochem. 58*:575-606 (1989)). In addition, HBGF-1 is chemotactic for endothelial cells, fibroblasts and astroglial cells. Both HBGF-1 and HBGF-2 bind to heparin, which protects these factors from proteolytic degradation. The array of biological activities exhibited by the HBGFs suggests that they play an important role in wound healing.

Growth factors are, therefore, potentially useful for specifically promoting wound healing and tissue repair. The addition of exogenous growth factors to a wound has been shown to increase the rate at which the wound is closed, the number of cells in the healing area, the growth of blood vessels, the total rate of deposition of collagen, and strength of the scar (see, *e.g.,* Carter *et al.,* in *Growth Factors and Other Aspects of Wound Healing: Biological and Clinical Implications,* Alan R. Liss, Inc., pp. 303-317 (1988)). Platelet-derived wound healing formula (PDWHF), a platelet extract which is in the form of a salve or ointment for topical application, has been described (see, *e.g.,* Knighton *et al., Ann. Surg. 204*:322-330 (1986)).

Experiments designed to test the efficacy of topical application of growth factors, however, have yielded inconsistent results. PDGF, IGF-1, EGF, TGF-β and fibroblast growth factor (FGF or HBGF) applied separately to a model wound system had little effect on the regeneration of connective tissue or epithelium in the wounds (Lynch *et al., J. Clin. Invest. 84*:640-646 (1989)). Of the factors tested, TGF-β stimulated the greatest response alone. A combination of factors, such as PDGF-2 homodimer and IGF-1 or TGF-α, however, produced "a dramatic increase in connective tissue regeneration and epithelialization." However, others have reported that application of individual growth factors to a wound stimulated wound healing (see, *e.g.,* Tsuboi *et al., J. Exp. Med. 172:*245-251 (1990)).

The reasons for such inconsistent results are not known, but might be the result of difficulty in applying growth factors to a wound in a manner in which they can exhibit their normal array of biological activities. Because of such inconsistent results, the role played in stimulating wound healing by exogenously applied growth factors is not clear. Further, a means by which growth factors might be applied to internal wounds to produce prolonged contact between the wound and the growth factor(s) is not presently known.

Surgical adhesives and tissue sealants which contain plasma proteins are known and are used for sealing internal and external wounds in order to reduce blood loss and maintain hemostasis. Such sealants typically contain blood clotting factors and other blood proteins. For example, European Patent 0,068,047 describes an anhydrous powder that is derived from an enriched plasma fraction that contains fibrinogen, a fibrinolysis inhibitor, and thrombin or prothrombin. It can only be applied as a powder because a clot forms as soon as water is added. Australian Patent AU-A-75097/87 describes a one-component adhesive, which contains an aqueous solution of fibrinogen, factor XIII, a thrombin inhibitor, such as antithrombin III, prothrombin factors, calcium ions, and, if necessary, a plasmin inhibitor. Stroetmann, U.S. Patent Nos. 4,427,650 and 4,427,651, describes the preparation of an enriched plasma derivative in the form of a powder that contains fibrinogen, thrombin and/or prothrombin, and a fibrinolysis inhibitor, and may also contain other ingredients, such as a platelet extract. Rose *et al.,* U.S. Patent Nos. 4,627,879 and 4,928,603 disclose methods for preparing cryoprecipitated suspensions that contain fibrinogen and Factor XIII and their use to prepare a fibrin glue. JP 1-99565 discloses a kit for the preparation of fibrin adhesives. Alterbaum, R. (U.S. Patent No. 4,714,457) and Morse *et al.* (U.S. Patent No. 5,030,215) disclose methods to produce autologous fibrin glue. In addition, improved fibrin glue delivery systems have been disclosed (Miller *et al.,* U.S. Patent No. 4,932,942; Morse *et al.,* PCT Application WO 91/09641).

Fibrin glue, which is also called fibrin sealant, is primarily formulated for clinical topical application and is used to control bleeding and promote wound healing. The clinical uses of fibrin glue have recently been reviewed (Gibble *et al., Transfusion 30:*741-747 (1990); Lerner *et al., J. Surg. Res. 48:*165-181 (1990)). Fibrin glues are commercially available. For example, IMMUNO AG (Vienna, Austria) and BEHRINGWERKE AG (Germany) presently have such products on the market (see, *e.g.,* U.S. Patent Nos. 4,377,572 and 4,298,598, which are owned by IMMUNO AG). BAXTER TRAVENOL (Waukegan, IL) has recently developed a fibrin glue.

Fibrin glues are prepared from plasma. The precise components of each fibrin glue are a function of the particular plasma fraction used as a starting material. Typically fibrin glue contains a mixture of proteins that, upon mixing with thrombin, form a clot. For example, fibrin glue can be prepared from plasma by cryoprecipitation followed by fractionation, to yield a composition that forms a sealant or clot upon mixture with thrombin or an activator of thrombin. Fractionation of plasma components can be effected by standard protein purification methods, such as ethanol, polyethylene glycol, and ammonium sulfate precipitation, and ion exchange, and gel filtration chromatography.

Fibrin glues generally include a fibrinogen concentrate, which contains fibronectin, Factor XIII, von Willebrand factor, and dried human or bovine thrombin. It is prepared in lyophilized form and is mixed with a solution of calcium chloride immediately prior to use. Upon mixing, the components coagulate on the tissue surface and form a clot that includes cross-linked fibrin. Factor XIII, which is present in the fibrinogen concentrate, catalyzes the cross-linking. Fibrin glue, by sealing tissues face to face, prevents air or fluid leaks, and thereby induces hemostasis. By virtue of the ability to maintain hemostasis and reduce blood loss, fibrin glue promotes wound healing. It does not, however, possess true wound healing properties. Because fibrin glue is suitable for both internal and external injuries and is useful to maintain hemostasis, it would be desirable to enhance its wound healing properties.

Fibrin glue with a fibrinogen concentration of approximately 39 g/l and a thrombin concentration of 200-600 U/ml produced wounds with significantly increased stress, energy absorption and elasticity values (Byrne *et al., Br. J. Surg. 78*:841-843 (1991)). In addition, fibrin glue did not affect ectopic osteo-chondrogenesis induced by bone morphogenetic protein in mice (Hattori, T., *Nippon Seikeigeka Gakkai. Zasshi. 64*:824-834 (1990)).

WO-A-9109573 and WO-A-8600526 both describe compositions comprising fibrin. In WO-A-9109573 the tissue sealant may alternatively comprise fibrimogen. However, none of these compositions contains a growth factor. EP-A-0312208 discloses a gel formulation for wound healing comprising growth factors such as TGF or TGF. But there is no disclosure of such a composition also comprising fibrinogen or fibrin glue.

Artificial vascular prostheses, which are frequently made out of polytetrafluoroethylene (PTFE), are used to replace diseased blood vessels in humans and other animals. To maximize patency rates and minimize the thrombogenicity of these vascular prostheses various techniques have been used including seeding of nonautologous endothelial cells onto the prothesis. Various substrates which adhere both to the graft and endothelial cells have been investigated as an intermediate substrate to increase endothelial cell seeding. These substrates include preclotted blood (Herring *et al., Surgery 84*:498-504 (1978)), fibrin glue (Rosenman *et al., J. Vasc. Surg. 2*:778-784 (1985); Schrenk *et al., Thorac. Cardiovasc. Surg. 35*:6-10 (1986); Köveker *et al., Thorac. Cardiovasc. Surgeon 34:* 49-51 (1986); Zilla *et al, Surgery 105*:515-522 (1989)), fibronectin (see, e.g., Kesler *et al., J. Vasc. Surg. 3:*58-64 (1986); Macarak *et al*., *J. Cell Physiol. 116:*76-86 (1983); Ramalanjeona *et al., J. Vasc. Surg. 3*:264-272 (1986)), or collagen (Williams *et al., J. Surg. Res. 38*:618-629 (1985)). However, one general problem with these techniques is that nonautologous cells are used for the seeding, (see, e.g., Schrenk *et al., supra)* thus, raising the possibility for tissue rejection, and further that a confluent endothelium, if ever established, requires months. As a result of this delay, there is a high occlusion rate (see, e.g., Zilla *et al*., *supra).* In addition, endothelial cell damage and cell loss occur due to shear stress (*Id*.).

In bone nonunion defects there is a minimal gap above which no new bone formation occurs naturally. Clinically, the treatment for these situations is bone grafting. The source of bone autografts is usually limited and the use of allogenic bones involves a high risk of viral contamination. Because of this situation, the use of demineralized, virally inactivated bone powder is an attractive solution. The bone demineralized matrix (DBM) contains growth factors and bone morphogenic proteins (BMP's) that are able to induce formation of new bone in animal models when the DBM is held in place (im, ip or sc) with a variety of materials including fibrin glue (Schwarz *et al., Clin. Orthopaed. Related Res. 238:*282-287 (1989)). From the immunogenic point of view, DBM is species specific and the higher the animal is in the evolutionary scale the less osteoinductive it is. Human DBM has, for this reason, limited applications.

GB-A-2137209 discloses a bone-repair composition comprising cells in a gel containing fibrinogen, and optionally growth factors (IGF, FGF). But the fibrinogen concentration is from about 25 to 80 mg/ml.

Purified BMP's of natural (Mark *et al., Plastic Reconstr. Surg. 86:*623-630 (1990)) or recombinant (D'Alessandro *et al., J. Cell. Biochem. 15F*:Q105, Abstract (1990)) origin have osteoinductive effects in animal models when delivered by a variety of means including whole blood clots (Wang *et al., J. Cell. Biochem. 15F*:Q20 Abstract (1990)) or fibrin glue (Hatori, *Nippon. Seikeigeka. Gakkai. Zasshi. 64*:824-834 (1990)).

It is therefore an object of this invention to provide a growth factor-supplemented tissue sealant, such as fibrin glue, that has the ability to promote: wound healing, such as of the skin and bone; endothelialization of vascular protheses; and the *in vitro* proliferation and/or differentiation of animal cells, such as endothelial cells and fibroblasts.

A further object of this invention is the use of a composition as claimed for the manufacture of a pharmaceutical for delivering growth factor(s) to wounded tissue, to vascular prostheses and to animal cells, such as endothelial cells and fibroblasts, cultured *in vitro,* and to maintain the growth factor(s) in contact with the injured tissue, vascular prostheses and animal cells for a prolonged period of time, whereby accelerated wound healing, endothelialization, or proliferation and differentiation of the animal cells results therefrom.

### SUMMARY OF THE INVENTION

A tissue sealant, such as fibrin glue, that contains an effective concentration of at least one growth factor is provided. This growth factor-supplemented tissue sealant promotes wound healing, endothelialization of vascular prostheses and the *in vitro* proliferation and differentiation of animal cells, especially endothelial cells and fibroblasts. Methods of using the growth factor-supplemented tissue sealants are also provided for accelerating or otherwise promoting wound healing, producing endothelialization of vascular prostheses and the *in vitro* proliferation and differentiation of animal cells, especially endothelial cells and fibroblasts.

The growth factor-supplemented tissue sealants of this invention may be particularly useful for promoting healing in wounds that do not readily heal, such as wounds in diabetic individuals, and for delivering growth factors to and providing a medium for prolonged contact between an internal wound site and the growth factor(s). For example, the growth factor-supplemented tissue sealants may be applied to broken bones or gastric ulcers and other such internal wounds in order to accelerate or promote healing thereof.

One object of this invention is to provide a mixture of fibrin glue, human DBM and purified BMP's for clinical purposes. This mixture provides a matrix that allows the cellular components of the human body to migrate and fill the gap of a nonunion defect where the osteoinduction needs to occur. The matrix composition in terms of proteins (such as fibrinogen and Factor XIII), enzymes (such as thrombin and plasmin) bone morphogenic proteins, growth factors and DBM are adequately formulated to optimize the longevity of this temporal scaffolding structure. All the mixture components are biodegradable but during osteogenesis the mixture provides a non-collapsible scaffold that can determine the shape of the newly formed bone. Soft tissue collapse into the bony nonunion defect, which is a problem in bone reconstructive surgery, will thus be avoided.

In a preferred embodiment, an effective concentration of HBGF-1 is added to a fibrin glue in order to provide a growth factor-supplemented tissue sealant that possesses the ability to accelerate and/or promote wound healing. In another preferred embodiment, an effective amount of a platelet-derived extract is added to a fibrin glue. In other preferred embodiments, an effective concentration of a mixture of at least two growth factors are added to fibrin glue and an effective amount of the fibrin glue which contains the growth factor(s) is applied to the wounded tissue.

In addition to growth factors, drugs, polyclonal and monoclonal antibodies, and other compounds may be added to the tissue sealant such as compounds that: accelerate wound healing; mediate or enhance the activity of the growth factor in the tissue sealant; and/or compounds that inhibit or interfere with tissue sealant components which inhibit the activities of the growth factor in the tissue sealant.

In one embodiment of the present invention is provided a composition that promotes the healing of wounds, especially wounds of the skin and bone, comprising a tissue sealant and an effective concentration of at least one growth factor, wherein the concentration is effective in accelerating or otherwise promoting wound healing.

In another embodiment of the present invention is provided the use of a composition that contains a tissue sealant and an effective concentration of at least one growth factor for promoting the healing of wounds, especially wounds of the skin and bone.

In another embodiment of the present invention is provided a composition that promotes the endothelialization of a vascular prosthesis, comprising a tissue sealant and an effective concentration of at least one growth factor, wherein the concentration is effective in accelerating or otherwise promoting the endothelialization of the vascular prosthesis.

In another embodiment of the present invention is provided the use of a composition that contains a tissue sealant and an effective concentration of at least on growth factor, applied to a vascular prosthesis, for the manifacture of a medicament for promoting the endothelialization of the vascular prosthesis.

In another embodiment of the present invention is provided a composition that promotes the *in vitro* proliferation and/or differentiation of animal cells, comprising a tissue sealant and an effective concentration of at least one growth factor, wherein said concentration is effective in accelerating or otherwise promoting the *in vitro* proliferation and/or differentiation of the animal cells.

In another embodiment of the present invention is provided a process for promoting the *in vitro* proliferation and/or differentiation of animal cells, comprising placing said cells onto and/or into a tissue sealant which contains an effective concentration of at least one growth factor, wherein the concentration is effective in accelerating or otherwise promoting the *in vitro* proliferation and/or differentiation of said cells.

The present invention has several advantages over the previously used compositions and methods. The first advantage is that the growth factor-supplemented tissue sealant of the present invention is biodegradable and can be reabsorbed by body tissues.

A second advantage is that the present invention provides a good way to effectively deliver growth factors within the body to a wound or other location. It is now believed that growth factor receptors must be occupied for at least 12 hours for a maximum biological effect to be produced by the growth factor. Previously, there was no way to do this to an internal location within the body. The present invention allows for prolonged contact between the growth factor and its receptors to occur, and thus allows for the production of strong biological effects.

A third advantage of the present invention is that the total protein and fibrinogen concentrations are more dilute than those previously used. This produces a fibrin glue that is about one fifth (1/5) as concentrated as commercially available fibrin glue. As a result of its less concentrated nature, animal cells can migrate into and through, and grow in the growth factor-supplemented fibrin glue of the present invention. This aids engraftment of the cells to neighboring tissues and prostheses. The fibrin glue which is commercially available is too dense to allow cells to migrate into and through it, and to grow in it. Instead, animal cells must migrate around or digest commercially available fibrin glue.

A fourth advantage of the present invention is that because of its liquid nature it can cover surfaces more thoroughly and completely than previously available delivery systems. This is especially important for the use of the present invention in the endothelialization of vascular prostheses because the growth factor-supplemented fibrin glue will coat the interior, exterior and pores of the vascular prosthesis. As a result of this, engraftment of autologous endothelial cells will occur simultaneously along the whole length of the vascular prosthesis, thereby decreasing its thrombogenicity and antigenicity. With previously used tissue sealants, engraftment started at the ends of the vascular prosthesis and proceeded into the interior of the same, thus allowing a longer period for thrombogenicity and antigenicity to develop. Previously used tissue sealants for vascular prostheses also primarily were seeded with nonautologous cells which could be rejected by the body and could be easily washed off by the shearing force of blood passing through the prosthesis.

### BRIEF DESCRIPTION OF THE FIGURES

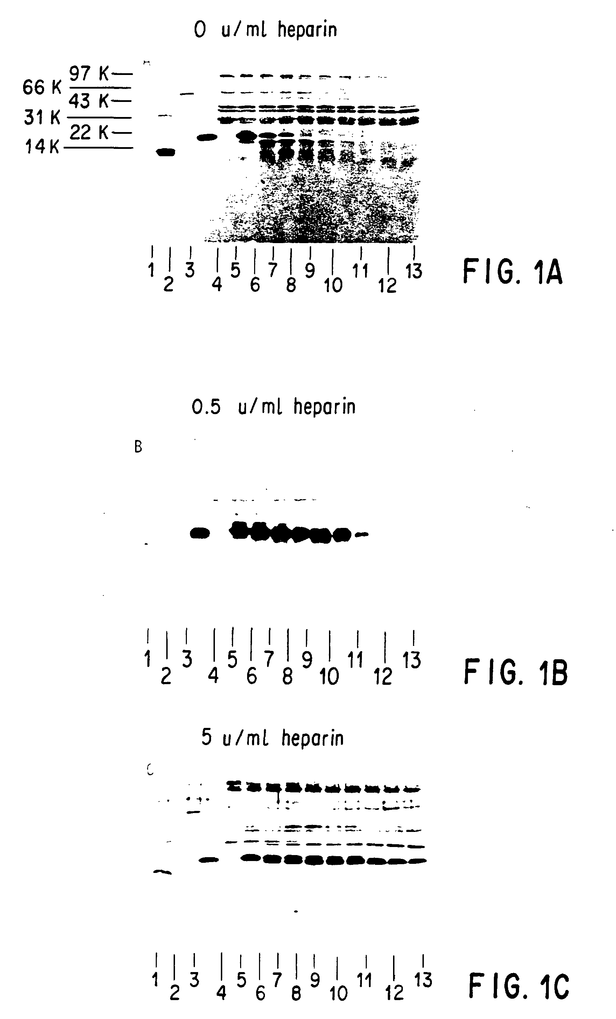

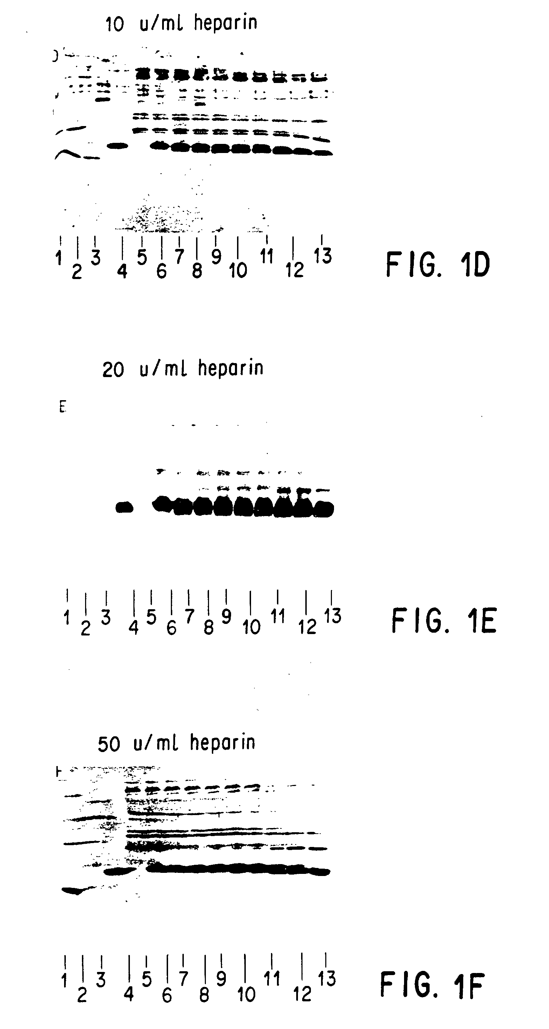

Fig. 1 shows Western blots of gels on which samples containing HBGF-1β had been incubated with 250 U/ml thrombin in the presence of increasing concentrations of heparin. Solutions containing HBGF-1β (10 µg/ml), thrombin (250 µg/ml), and increasing concentrations of heparin (0, 0.5, 5, 10, 20, and 50 units/ml) were incubated at 37°C for 72 hours. Aliquots were periodically removed from each of the incubating mixtures and were loaded onto 8% SDS polyacrylamide gels that were prepared and run as described by Laemmli *(Nature 227:*680 (1970)). The gel was then electroblotted onto nitrocellulose and the band corresponding to HBGF-1β was identified using an affinity-purified polyclonal rabbit antiserum to HBGF-1β.

The concentrations of heparin in the incubating mixtures were: panel A) 0 units/ml; panel B) 0.5 units/ml; Panel C) 5 units/ml; panel D) 10 units/ml; panel E) 20 units/ml; and panel F) 50 units/ml. In the gels pictured in each of panels A-F, each lane contains the following: lane 1 contains SDS-PAGE low molecular weight standards; lane 2 contains biotinylated standards; lane 3 contains 10 µg/ml HBGF-1β, lane 4 contains 250 Units/ml thrombin; and lanes 5-13 contain samples removed from the incubating mixtures at times 0, 1, 2, 4, 6, 8, 24, 48, and 72 hours.

Fig. 2 shows the incorporation of ³H-thymidine as a function of relative HBGF-1β concentration. Samples of the HBGF-1β were incubated, as described in Figure 1 and Example 2, in the presence of 250 U/ml thrombin and 5 U/ml heparin for 0 hour, 24 hours or 72 hours. Dilutions of these samples were then added to NIH 3T3 cells, which were plated as described in Example 3. CPM is plotted v. HBGF-1 concentration.

Fig. 3. Typical pattern of human umbilical vein endothelial cells after 7 days' growth on fibrin glue supplemented with 100 ng/ml of active, wild-type FGF-1. Note the large number of cells and their elongated shape. Compare with the paucity of cells grown on unsupplemented fibrin glue (Figure 5).

Fig. 4. Typical pattern of human umbilical vein endothelial cells after 7 days' growth on fibrin glue supplemented with 10 ng/ml of active, wild-type FGF-1. Note the large number of cells and their elongated shape. Compare with the paucity of cells grown on unsupplemented fibrin glue (Figure 5).

Fig. 5. Typical pattern of human umbilical vein endothelial cells after 7 days' growth on unsupplemented fibrin glue. Note the small number of cells, which indicates a slower proliferation rate, compared to the number in Figures 3 and 4.

Fig. 6. Typical pattern of human umbilical vein endothelial cells after 7 days' growth on fibrin glue supplemented with 100 ng/ml of inactive, mutant FGF-1. Note the small number of cells, which indicates a slower proliferation rate, compared to the number in Figures 3 and 4.

Fig. 7. Typical pattern of human umbilical endothelial cells 24 hours after having been embedded in fibrin glue at a concentration of 10⁵ cells per ml of glue. The protein and thrombin concentrations of the glue were 4 mg/ml and 0.6 NIH units/ml, respectively. Note, their elongated, multipodial morphology and that they formed a cellular network where they came in contact with each other. Compare with the cobblestone shape of similar cells grown in fibronectin (Fig. 9.)

Fig. 8. Typical pattern of human umbilical endothelial cells 48 hours after having been embedded in fibrin glue at a concentration of 10⁵ cells per ml of glue. The culture conditions were as described in Fig. 7. Note the further accentuated, elongated and multipodial morphology and increasing development of cellular networks. Compare with the cobblestone shaped cells grown in fibronectin (Fig. 10) and note the lack of a cellular network in the latter.

Fig. 9. Typical pattern of human umbilical endothelial cells 24 hours after having been cultured on a surface coated with fibronectin. Note, the cobblestone shape of the cells and the lack of cellular networks. Compare to Figure 7.

Fig. 10. Typical pattern of human umbilical endothelial cells 48 hours after having been cultured in a commonly used film of fibronectin. Note, the cobblestone shape of the cells and the lack of cellular networks. Compare to Figure 8.

Figure 11. Micrographs of cross sections of PTFE vascular grafts that were explanted from dogs after 7 days (panels A, C, E) or 28 days (panels B, D, F). Prior to implantation, the grafts were either untreated (A and B), coated with fibrin glue alone (C and D), or coated with fibrin glue supplemented with heparin and HBGF-1 (E and F).

Figure 12. Scanning electron micrographs of the inner lining of the vascular grafts described in Figure 11 after 28 days of implantation. The grafts were either untreated (G), coated with fibrin glue alone (H), or coated with fibrin glue supplemented with heparin and HBGF-1 (I).

### DESCRIPTION OF THE PREFERRED EMBODIMENTS

### Definitions

Unless defined otherwise, all technical and scientific terms used herein have the same meaning as is commonly understood by one of skill in the art to which this invention belongs.

As used herein, a wound includes any damaged tissue in a living organism. The tissue may be an internal tissue, such as the stomach lining or a broken bone, or an external tissue, such as the skin. It may be a soft tissue, such as the spleen, or a hard tissue, such as bone. The wound may have been caused by any agent, including traumatic injury, infection or surgical intervention.

As used herein, a tissue sealant is a composition prepared from plasma proteins that, upon application to a wound, forms a dot, thereby sealing the wound, reducing blood loss and maintaining hemostasis. Tissue sealants, such as a fibrin glue, are known to those of skill in the art.

As used herein, such sealants include any tissue sealant or adhesive that, without substantial modification, can serve as a carrier vehicle for the delivery of a growth factor or mixture of growth factors to a wound site and that, by virtue of its adhesive or adsorptive properties, can maintain contact with the site for a time sufficient for the growth factor-supplemented tissue sealant to accelerate wound healing.

As used herein, a growth factor-supplemented tissue sealant is a tissue sealant to which at least one growth factor, or other compound that accelerates, promotes or improves wound healing, has been added at a concentration that is effective for accelerating, promoting or improving wound healing. The growth factor-supplemented tissue sealants may also contain additional components, including drugs, antibodies, anticoagulants and compounds that: 1) potentiate, stimulate or mediate the biological activity of the growth factor(s) in the tissue sealant; or 2) decrease the activities of components of the growth factor-supplemented tissue sealant which would inhibit or destroy the biological activities the growth factor(s) in the sealant.

As used herein, a potentiating compound is a compound that mediates or otherwise increases the biological activity of a growth factor in the tissue sealant. Heparin is an example of a compound that potentiates the biological activity of HBGF-1.

As used herein, an inhibiting compound is a compound that inhibits, interferes with, or otherwise destroys a deleterious activity of a component of the tissue sealant that would interfere with or inhibit the biological activity of a growth factor or factors in the tissue sealant. Inhibiting compounds may exert their effect by protecting the growth factor from degradation. An inhibiting compound does not, however, inhibit any activities that are essential for the wound healing properties of the growth factor-supplemented fibrin glue.

As used herein, a growth factor includes any soluble factor that regulates or mediates cell proliferation, cell differentiation, tissue regeneration, wound repair and/or any developmental or proliferative process. The growth factor may be produced by any appropriate means including extraction from natural sources, production through synthetic chemistry, production through the use of recombinant DNA techniques and any other techniques, including virally inactivated, growth factor(s)-rich platelet releasate, which are known to those of skill in the art.

As used herein, HBGF-1, which is also known to those of skill in the art by alternative names, such as endothelial cell growth factor (ECGF) and acidic FGF, refers to any biologically active form of HBGF-1, including HBGF-1β, which is the precursor of HBGF-1α and other truncated forms, such as FGF. U.S. Patent No. 4,868,113 to Jaye *et al.,* herein incorporated by reference, sets forth the amino acid sequences of each form of HBGF. HBGF-1 thus includes any biologically active peptide, including precursors, truncated or other modified forms, that exhibit the biological activities, or a subset thereof, of HBGF-1.

Other growth factors may also be known to those of skill in the art by alternative nomenclature. Accordingly, reference herein to a particular growth factor by one name also includes any other names by which such factor is known to those of skill in the art and also any biologically active precursors, truncated or otherwise modified, but biologically active, forms thereof.

As used herein, biological activity refers to one or all of the activities that are associated with a particular growth factor *in vivo* and *in vitro.* Generally, a growth factor exhibits several activities, including mitogenic activity (the ability to induce or sustain proliferation) and also non-mitogenic activities, including the ability to induce or sustain differentiation and/or development. In addition, growth factors are able to recruit or attract particular cells from which the prcliferative and developmental processes proceed. For example, under appropriate conditions HBGF-1 can recruit endothelial cells and direct the formation of vessels therefrom. By virtue of this activity, growth factor-supplemented tissue sealants may thereby provide a means to enhance blood flow and nutrients to specific sites.

### Preparation of Growth Factor-Supplemented Tissue Sealant

As a first step when practicing any of the embodiments of the invention disclosed herein, the growth factor and tissue sealant must be selected. Each may be prepared by methods known to those of skill in the art or may be purchased from a supplier thereof. In a preferred embodiment, growth factor-supplemented fibrin glue is prepared.

### Preparation of Tissue Sealants.

Any tissue sealant, such as a fibrin glue, may be used in this invention. For example, fibrin glues which are well known to those of skill in the art (see, *e.g.,* U.S. Patent Nos. 4,627,879, 4,377,572 and 4,298,598, herein incorporated by reference) may be purchased from a supplier or manufacturer thereof, such as IMMUNO AG (Vienna, Austria) and BEHRINGWERKE AG (Germany). Alternatively, fibrin glue can be readily prepared from plasma or plasma components. The particular composition of the selected tissue sealant is not critical as long as it functions *in vivo* as a tissue sealant and can serve as a medium to contact the growth factor(s) with the wounded tissue or vascular graft, whereby wound healing or endothelialization is promoted or accelerated.

For the experiments exemplified herein, fibrin glue was prepared from the cryoprecipitate from fresh frozen plasma. The components of the fibrin glue that were used as a vehicle for a growth factor included: fibrinogen; fibronectin; factor XIII; and von Willebrand Factor.

In a preferred embodiment of this invention, the total protein concentration is from about 5 to 150 mg/ml of solution used to prepare the fibrin glue. In a more preferred embodiment, the total protein concentration in this preparatory solution is from about 7 to 35 mg/ml. In the most preferred embodiment, the total protein concentration in this preparatory solution is from about 17 to 28 mg/ml.

In a preferred embodiment of this invention, the fibrinogen concentration is from about 4 to 135 mg/ml of solution used to prepare the fibrin glue. In a more preferred embodiment, the fibrinogen concentration in this preparatory solution is from about 6 to 30 mg/ml. In the most preferred embodiment, the fibrinogen concentration in this preparatory solution is from about 15 to 25 mg/ml.

Although the growth factor concentration will vary with the growth factors used, in a preferred embodiment of this invention, the growth factor concentration is from about 1 ng/ml to 1 mg/ml of fibrin glue. In a more preferred embodiment, the growth factor concentration is from about 1 µg/ml to 100 µg/ml of fibrin glue. In the most preferred embodiment, the growth factor concentration is from about 5 µg/ml to 20 µg/ml of fibrin glue. The amount of the growth factor(s) to add can be empirically determined by testing various concentrations and selecting that which is effective for the intended purpose, and the site of application.

### Preparation of Growth Factors.

The growth factor(s), or mixture thereof, may be prepared by any method known to those of skill in the art or may be purchased commercially. Any growth factor may be selected including, but not limited to, for example, growth factors that stimulate the proliferation of certain cell types, such as endothelial cells, fibroblasts, epithelial cells, smooth muscle cells, hepatocytes, and keratinocytes, and/or growth factors which inhibit the growth of the same cell types, and also smooth muscle cells. Such selection may be dependent upon the particular tissue site for which the growth factor-supplemented tissue sealant will be applied. For example, an EGF-supplemented tissue sealant may be preferred for application to wounds in the eye and for treating gastric ulcers while an osteogenin-supplemented tissue sealant may preferred for application to bone fractures and breaks in order to accelerate or promote healing thereof.

In another preferred embodiment HBGF-1β was prepared and added to a fibrin glue. HBGF-1β, or HBGF-1α, or any other active form of HBGF-1, can be purified from natural sources, from genetically engineered cells that express HBGF-1 or a derivative thereof, or by any method known to those of skill in the art.

HBGF-1β has been prepared using recombinant DNA methodology (Jaye *et al*., U.S. Patent No. 4,868,113; Jaye *et al., J*. *Biol*. *Chem. 262*:16612-16617 (1987)). Briefly, DNA encoding HBGF-1β was cloned into a prokaryotic expression vector, a pUC9 derivative, and expressed intracellularly in *E*. *coli.* The expressed peptide was then released from the cells by pressure, using a cell disrupter operated on high compression-decompression cycles. After disruption, cell debris was removed by filtration and HBGF-1β was purified from the supernatant using standard methods of protein purification including affinity chromatography on heparin Sepharose™ followed by ion-exchange chromatography on CM-Sepharose™.

In addition to HBGF-1, described above, other growth factors that may be added to the fibrin glue include, but are not limited to, HBGF-2, IGF-1, EGF, TGF-β, TGF-α, any platelet-derived growth factor or extract, and mixtures of any growth factors. For example, platelet-derived extracts, which serve as rich sources of growth factors, may be added to the tissue sealant in addition to or in place of other growth factors, such as HBGF-1.

In a preferred embodiment, a platelet-derived extract, prepared by any method known to those of skill in the art, is added to a tissue sealant. Such an extract has been prepared from plasma reduced platelets for use with fibrin glue.

PDWHF may be prepared and added to fibrin glue (Knighton *et al., Ann. Surg. 204*:322-330 (1986)). Briefly, to prepare PDWHG, blood is drawn into anticoagulant solution and platelet-rich plasma is prepared by refrigerated centrifugation. The platelets are isolated and stimulated with thrombin, which releases the contents of the alpha granule contents. The platelets are removed and an effective concentration of the remaining extract is added to a tissue sealant.

### Additional Components of Growth Factor-Supplemented Tissue Sealants.

Since they are essentially plasma fractions, the tissue sealants contemplated for use with growth factors contain numerous components, some of which may interfere with the biological activity of the selected growth factor(s). For example, thrombin, which is an essential component of fibrin glue, can act as a proteolytic enzyme and specifically cleave HBGF-1β. Therefore, it may be necessary to include additional compounds, such as protease or other inhibitors, that protect the selected growth factor(s) from the action of other components in the tissue sealant which inhibit, interfere with or destroy the biological activity of growth factors.

Selection of the particular inhibiting compound(s) may be empirically determined by using methods, discussed below, that assess the biological activity of the growth factor(s) in the tissue sealant. Methods to assess biological activity are known to those of skill in the art.

In addition, in order for certain growth factors to exhibit their biological activities, it may be necessary to include compounds that potentiate or mediate the desired activity. For example, heparin potentiates the biological activity of HBGF-1 *in vivo* (see, *e.g.,* Burgess *et al., Annu. Rev. Biochem. 58*:575-606 (1989)).

The growth-factor supplemented sealants of the present invention may also contain other compounds such as drugs, other chemicals, and proteins. These may include, but are not limited to: antibiotics; anticoagulants, such as activated protein C, heparin, prostracyclin (PGI₂), antithrombin III, ADPase, and plasminogen activator; steroids to inhibit inflammation, such as dexamethasone; cardiovascular drugs, such as calcium channel blockers; chemoattractants; and local anethetics such as bupivacaine. These supplemental compounds may also include polyclonal, monoclonal or chimeric antibodies, or functional derivatives or fragments thereof. They may be antibodies which, for example, inhibit smooth muscle proliferation, such as antibodies to PDGF, and/or TGF-β, or the proliferation of other undesirable cell types within and about the area treated with the tissue sealant. These antibodies can also be useful in situations where anti-cancer, anti-platelet or anti-inflammatory activity is needed. In general, any antibody whose efficacy or economy would be improved by site-directed delivery may benefit from this fibrin glue delivery system.

### Assays for Assessing the Wound Healing Properties of a Growth Factor-Supplemented Tissue Sealant

In order to ascertain whether a particular growth factor-supplemented tissue sealant accelerates or otherwise enhances wound healing and to select optimal concentrations of the growth factor(s) to do the same, the composition is tested by any means known to those of skill in the art for its ability to accelerate or otherwise promote wound healing (see, *e.g.,* Tsuboi *et al., J. Exp. Med. 172*:245-251 (1990); Ksander *et al., J. Am. Acad. Dermatol. 22*:781-791 (1990); and Greenhalgh *et al., Am. J. Path. 136:*1235 (1990)). Any method including both *in vivo* and *in vitro* assays, by which the activity of the selected growth factor(s) in the tissue sealant composition can be assessed may be used. For example, the activity of HBGF-1β has been assessed using two independent *in vitro* assays. In the first, the proliferation of endothelial cells that had been suspended in a shallow fluid layer covering a plastic surface which had been impregnated with growth factor-supplemented fibrin glue was measured. In the second, the incorporation of ³H-thymidine in cultured fibroblasts in the presence of HBGF-1 was measured.

In an *in vivo* assay, fibrin glue that had been supplemented with HBGF-1β has been tested for its ability to promote healing *in vivo* using mice as a model system. In this method identical punch biopsies were performed in the dorsal region of the mice, which were then separated into test, treated control and untreated control groups. The wounds on the mice in the test group were treated with the growth factor-supplemented tissue sealant. The wounds on the mice in the treated control group were treated with unsupplemented sealant. The wounds in the untreated group were not treated. After a time sufficient for detectable wound healing to proceed, generally a week to ten days, the mice were sacrificed and the wounded tissue was microscopically examined to histologically assess the extent of wound repair in each group.

The ability of the growth factor-supplemented tissue sealant to induce cell proliferation and to recruit cells may also be assessed by *in vitro* methods known to those of skill in the art. For example, the *in vitro* assays, described above for measuring the biological activity of growth factors and described in detail in the Examples, may be used to test the activity of the growth factor in the tissue sealant composition. In addition, the effects of adding candidate inhibiting and/or potentiating compounds can also be assessed.

Generally, the necessity for adding inhibiting and/or potentiating compounds can be empirically determined. For example, in the experiments described below, the HBGF-1β in HBGF-1-supplemented fibrin glue was specifically cleaved in a stochastic manner, suggesting that a component of the fibrin glue preparation, most likely thrombin, was responsible. Heparin, which is known to bind to HBGF-1 and protect it from certain proteolytic activities, was added to the HBGF-1-supplemented fibrin glue. The addition of relatively low concentrations of heparin protected HBGF-1β from cleavage that would destroy its biological activity in the fibrin glue. Therefore, tissue sealant compositions that include HBGF-1 must include heparin or some other substance that inhibits the cleavage of HBGF-1 by thrombin or other proteolytic components of the fibrin glue.

Similarly, the ability of a selected inhibitor to protect a growth factor from degradation by tissue sealant components may be assessed by any method known to those of skill in the art. For example, heparin has been tested for its ability to inhibit cleavage of HBGF-1 by thrombin, which is an essential component of fibrin glue. To do so, mixtures of various concentrations of heparin and HBGF-1-supplemented fibrin glue have been prepared, and incubated for various times. The biological activity of HBGF-1 in the mixture has been tested and the integrity of such HBGF-1 has been ascertained using western blots of SDS gels. Relatively low concentrations, about a 1:1 molar ratio of heparin:HBGF-1, are sufficient to protect HBGF-1 from degradation in fibrin glue.

It can also be empirically determined whether a particular compound can be used to potentiate, mediate or enhance the biological activity of a growth factor or factors in a tissue sealant.

### Topical or Internal Application of the Growth Factor-Modified Tissue Sealant to an Internal or External Wound.

Prior to clinical use, the growth factor and tissue sealant, or the growth factor-supplemented tissue sealant is pasteurized or otherwise treated to inactivate any pathogenic contaminants, such as viruses. Methods for inactivating contaminants are well-known to those of skill in the art and include, but are not limited to, solvent-detergent treatment and heat treatment (see, *e.g.,* Tabor *et al., Thrombosis Res. 22:*233-238 (1981) and Piszkiewicz *et al., Transfusion 28*:198-199 (1988)).

The growth factor-modified tissue sealant is applied directly to the wounded tissue. Typically for external wounds it can be applied directly by any means, including spraying on top of the wound. It can also be applied internally, such as during a surgical procedure. When it is applied internally, such as to bones, the clot gradually dissolves over time.

The following examples are included for illustrative purposes only and are not intended to limit the scope of the invention.

### EXAMPLE 1

### Preparation of HBGF-1-Supplemented Fibrin Glue.

Fibrin glue prepared from the cryoprecipitate fraction of plasma was used. Plasma was prepared from human blood by centrifugation to pellet the red cells and to remove the platelets. The plasma was centrifuged and the cryoprecipitate was collected and fractionated to produce a fibrin glue.

An 800 ml culture of recombinant *E*. *coli* containing a plasmid that included DNA encoding HBGF-1β was prepared. After induction and culturing for 24 hours at 37°C, the cells were centrifuged and the supernatant discarded. The cell pellet was resuspended in 25 mls of 20 mM phosphate buffer, containing 0.15 M NaCl, pH 7.3. The suspended cells were disrupted with a cell disrupter and the cell debris was separated from the resulting solution by centrifugation at 5000 g for 20 min.

The pellet was discarded and the supernatant containing the solubilized HBGF-1β and other bacterial proteins was loaded onto a 2.6 cm diameter by 10 cm high column of Heparin-Sepharose™ (Pharmacia Fine Chemicals, Upsala, Sweden). The column was washed with 5 column volumes of 0.15 M NaCl in 20 mM phosphate buffer, pH 7.3, and then was eluted with a 0.15 M NaCl in 20 mM phosphate buffer to 2.0 M NaCl gradient.

The eluate was monitored by UV absorption at 280 nm. Three peaks of UV absorbing material eluted and were analyzed by SDS polyacrylamide gel electrophoresis. Peak number three electrophoresed as a single band at about 17,400 daltons and contained substantially pure HBGF-1β.

In order to further insure that the HBGF-1β was free of contaminating bacterial proteins, peak number three, which contained the growth factor activity, was dialyzed overnight against 20 mM histidine, 0.15 M NaCl, pH 7.5. Two mg of protein was loaded onto a 1 ml CM-Sepharose™ (Pharmacia, Upsala, Sweden) ion exchange column. The column was washed with 10 bed volumes (0.5 ml/min) of 20 mM histidine, 0.15 M NaCl, pH 7.5 and eluted with a gradient of 0.15 M NaCl to 1.0 M NaCl in 20 mM histidine, pH 7.5. The eluate was monitored by UV absorption at 280 nm and HBGF-1β was identified by SDS polyacrylamide gel electrophoresis.

One to 10 µg of the purified HBGF-1β was added to the fibrin glue.

### EXAMPLE 2

### Stability of HBGF-1 in Fibrin Glue.

It was necessary to add an ingredient to the fibrin glue that would inhibit or prevent the digestion of HBGF-1β by thrombin, which is a component of fibrin glue. Heparin, which adsorbs to HBGF-1, was selected and tested to determine whether it could protect HBGF-1 from digestion by thrombin and any other proteolytic components of the fibrin glue. The stability of HBGF-1 in the presence of increasing concentrations of heparin was assessed.

Solutions containing HBGF-1β (10 µg/ml), thrombin (250 µg/ml), and increasing concentrations of heparin (0, 0.5, 5, 10, 20, and 50 units/ml) were incubated at 37°C. Aliquots were periodically removed from the incubating solutions and were frozen and stored at -70°C for further testing.

After the incubation was complete, the samples were thawed and separated on 15% SDS polyacrylamide gels under reducing conditions according to the method of Laemmli (*Nature 227*:680 (1970)). The gel was then electroblotted onto nitrocellulose and the band corresponding to HBGF-1 was identified using an affinity-purified polyclonal rabbit antiserum to HBGF-1. The Western blots are shown in Fig. 1 on which the HBGF-1β band at 17.4 kD a can be seen. The results indicated that in the presence of concentrations of heparin as low as 5 U/ml, HBGF-1β was protected from digestion by thrombin. In addition, as described in Example 3, its biological activity was not altered.

### EXAMPLE 3

### The Biological Activity of HBGF 1β after Incubation in the Presence of Heparin and Thrombin.

The biological activity of HBGF-1 in the incubation mixture that contained 5 U/ml of heparin, and was described in Example 2, was measured using an ³H-thymidine incorporation assay with NIH 3T3 cells.

NIH 3T3 cells were introduced into 96 well plates and incubated at 37°C under starvation conditions in Dulbecco's Modified Medium (DMEM; GIBCO, Grand Island, New York) with 0.5% bovine calf serum (BCS; GIBCO, Grand Island, New York) until the cells reached 30 to 50% confluence. Two days later, varying dilutions of HBGF-1 from the samples prepared in Example 2 were added to each well without changing the medium. Diluent (incubation buffer) was added in place of growth factor for the negative controls and DMEM with 10% BCS, which contains growth factors needed for growth, was added in place of the HBGF-1 sample for the positive controls.

After incubation at 37°C for 18 hours, 0.25 µCi of ³H-thymidine, specific activity 6.7 µCi/mol, was added to each well and the incubation was continued at 37°C for an additional 4 hours. The plates were rinsed with phosphate-buffered saline (PBS) and fixed with 0.5 ml cold 10% trichloracetic acid (TCA) for 15 min at 4°C. The TCA was removed, the plates were rinsed with PBS and the acid-precipitable material was solubilized with 0.5 ml/well of 0.1 N sodium hydroxide for 1 hour at room temperature. The samples were transferred to scintillation vials and 10 ml of scintillation fluid (New England Nuclear, Aquasure™) was added per vial.

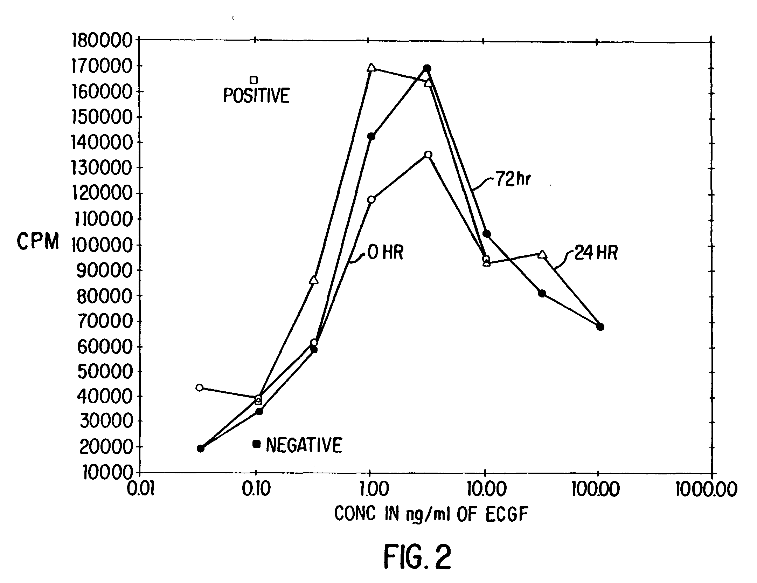

The results, which are shown in Figure 2, demonstrated that HBGF-1, which had been incubated in the presence of thrombin and heparin, retained its biological activity. The observed concentration dependence of thymidine incorporation was independent of incubation time and was typical of that expected for the dependence of the proliferation of cells as a function of growth factor concentration. Growth factors typically exhibit an optimal concentration at which cell proliferation is maximal.

The biological activity of HBGF-1 in the presence of thrombin and heparin was also measured by observing endothelial cell proliferation. The surfaces of petri dishes were impregnated with the HBGF-1 supplemented fibrin glue. A shallow layer of endothelial cells was added and the number of cells was measured. Over time the number of cells increased. In addition, the cells appeared to be organizing into vessels.

Therefore, HBGF-1 retains its biological activities in fibrin glue that includes heparin, which protects HBGF-1 from the degradative activity of thrombin and may also potentiate the biological activity of the HBGF-1 in the growth factor-supplemented fibrin glue.

### EXAMPLE 4

### HBGF-1 Diffuses Out of the Fibrin Glue Clot as a Function of Concentration and Time.

A fibrin glue clot was formed in a 5 ml plastic test tube by mixing 0.3 ml of the fibrinogen complex containing 10 U/ml heparin and thrombin and 40 mM CaC1₂. Four test tubes were set up as follows:
A: 0.5 U/ml thrombin and 10 µg/ml HBGF-1
B: 0.5 U/ml thrombin and 50 µg/ml HBGF-1
C: 5 U/ml thrombin and 10 µg/ml HBGF-1
D: 5 U/ml thrombin and 50 µg/ml HBGF-1.
The clot was covered with 0.2 M histidine buffer, pH 7.3. Thirty µl samples of the overlying buffer were removed from each tube every two hours and run on a western blot.

The results of the experiment demonstrated that HBGF-1 diffusion out of the clot is a function of time and its concentration in the clot, and that the concentration of thrombin in the clot does not affect the rate at which HBGF-1 is released from the clot.

### EXAMPLE 5

### Behavior of Human Umbilical Vein Endothelial Cells in Growth Factor-Supplemented Fibrin Glue

To study the *in vitro* effects of acidic fibroblast growth factor (FGF-1)-supplemented fibrin glue on human endothelial cells, suspensions of these cells were added to 10 cm diameter petri dishes that contained evenly spread layers of 2.5 ml of fibrin glue containing approximately 9 mg of fibrinogen per ml and 0.25 NIH units of thrombin per ml. The fibrin glue was supplemented in the following ways:
A. No added growth factor;
B. Supplemented with 100 ng/ml of active, wild-type FGF-1;
C. Supplemented with 100 ng/ml of inactive, mutant FGF-1, or
D. Supplemented with 10 ng/ml of active, wild-type FGF-1.
The cells seeded onto the fibrin glue layer were maintained for 7 days in DMEM containing 10% fetal calf serum.

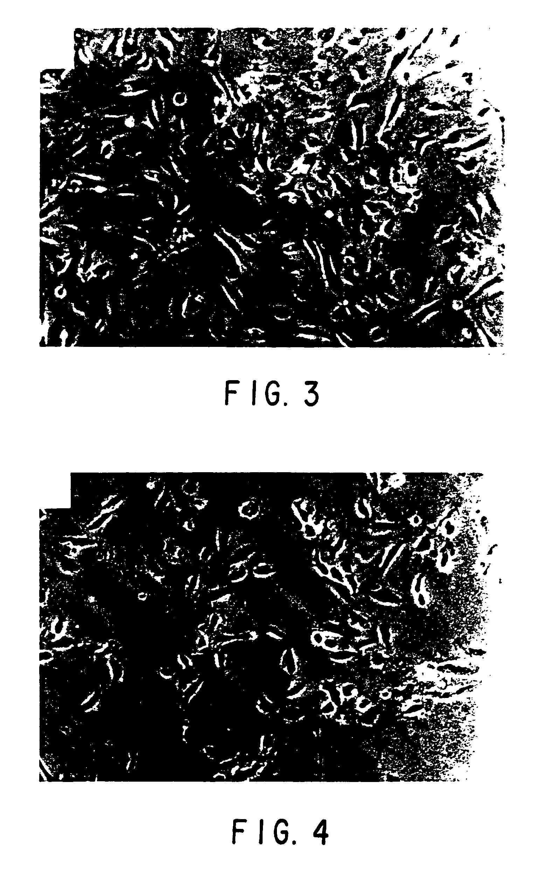

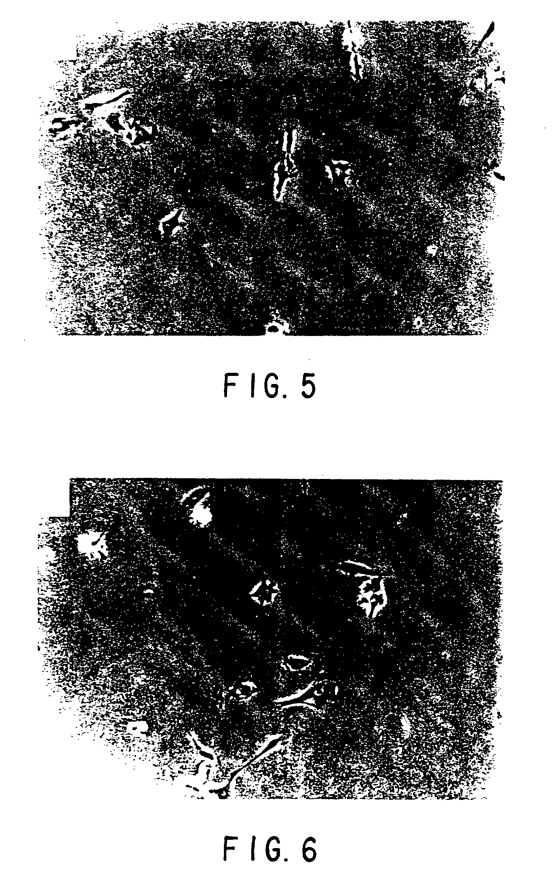

The cells became elongated and proliferated efficiently when in contact with fibrin glue supplemented with biologically active FGF-1 (Figures 3 and 4). In contact with unsupplemented fibrin glue (Figure 5) or with fibrin glue supplemented with biologically inactive FGF-1 (Figure 6), the cells become elongated but proliferated relatively slowly.

### EXAMPLE 6

### Behavior of Human Umbilical Vein Endothelial Cells in Fibrin Glue.

To study their growth, human umbilical endothelial cells, 10⁵ or more cells per ml, were embedded in fibrin glue, the protein concentration of which was 4 mg/ml. The concentration of thrombin in the fibrin glue was adjusted to 0.6 NIH units/ml. The culture medium used in all of the experiments was M199 (Sigma Chemical Co., St. Louis, MO) supplemented with 10% fetal bovine serum, 10 ug/ml streptomycin, 100 U/ml penicillin, 1 ng/ml acidic fibroblast growth factor and 10 U/ml heparin.

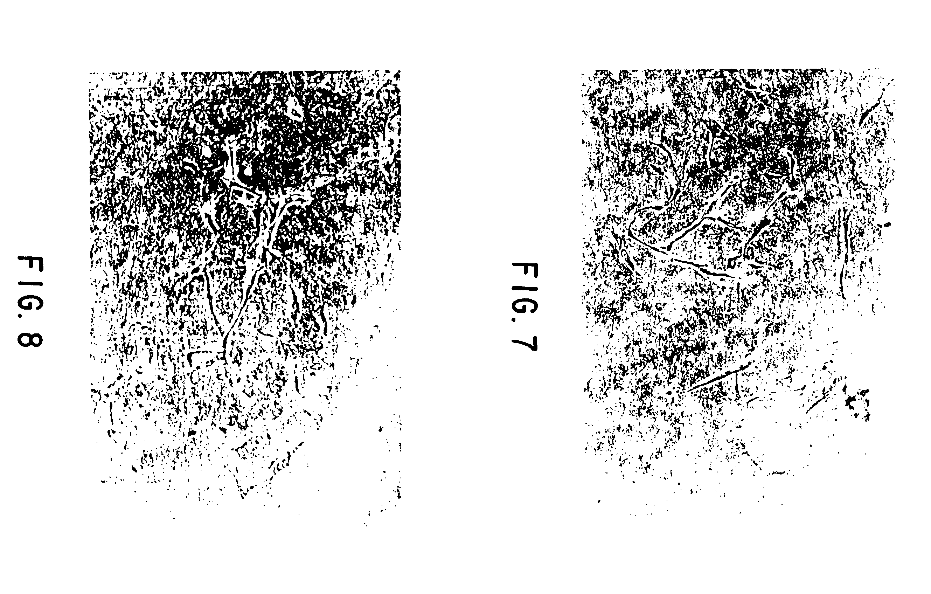

Within 24 hours in fibrin glue the cells became elongated, multipodial and formed a cellular network when they came in contact with each other (Fig. 7). This growth continued for at least 5 days. Figure 8 shows this situation at 48 hours.

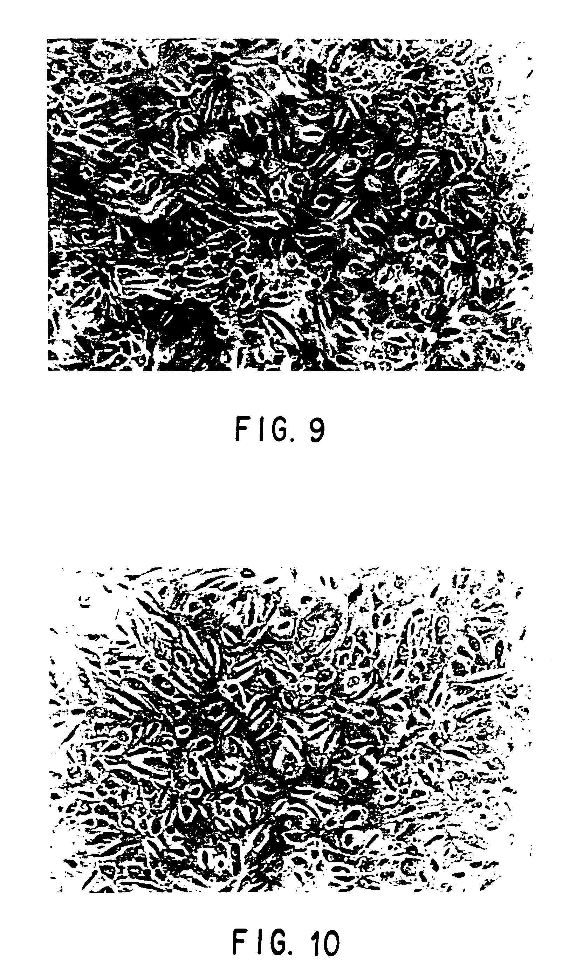

As a control, an identical cell suspension was cultured on a surface coated with fibronectin at 10 µg/cm². Control cells acquired a cobblestone shape and maintained this morphology for at least 5 days. Figures 9 and 10 show this situation at 24 and 48 hours, respectively.

### EXAMPLE 7

### Behavior of PMEXNEO-3T3-2.2 Cells In Fibrin Glue.

PMEXNEO-3T3-2.2 cells are fibroblast cells that contain a modified genome with the potential to express genetically engineered proteins. To determine the behavior of these cells in fibrin glue, 10⁵ cells per well were cultured under three conditions: (1) embedded in fibrin glue; (2) on the surface of fibrin glue; and (3) in the absence of fibrin glue (controls). The experiments were carried out in duplicate in 24-well plates in DMEM media (Sigma Chemical Co., St. Louis, MO) supplemented with 10% fetal calf serum (FCS). The fibrin glue protein concentration was 4 mg/ml. Identical experiments supplemented with 1.5% FCS were used as negative controls.

In the presence of media supplemented with 10% FCS, the cells in all 3 groups grew and became confluent. In the negative control experiments in which the media was supplemented with 1.5% FCS, the cells grew and survived for at least five days in the presence of fibrin glue, but not without it. However, their growth was faster in fibrin glue supplemented with 10% FCS than in that supplemented with 1.5% FCS. In the absence of fibrin glue, in the media supplemented with 1.5% FCS, the cells died within 48 hours. The criteria for survival was the ability of the tested cells to proliferate upon transfer to fresh media supplemented with 10% FCS.

### EXAMPLE 8

### Enhanced Endothelialization of Expanded Polytetrafluorethylene Grafts by Heparin Binding Growth Factor - Type 1 (HBGF-1) Pretreatment.

Two studies demonstrated that pretreatment of blood-contacting biomaterials with endothelial cell (EC) mitogens enhanced endothelialization. The first study examined the *in vivo* washout characteristics of HBGF-1, a known angiogenic factor, supplemented fibrin glue suspension applied to expanded polytetrafluorethylene (PTFE) grafts which were then implanted into rabbit aortas.

In general the modified fibrin glue was sterilely prepared by adding approximately 1 ng/cm² area of the inner and outer graft surfaces of human recombinant ¹²⁵I-HBGF-1, 20 µg/cm² porcine intestinal mucosal heparin, and 2.86 mg/cm² fibrinogen to 2.86 x 10⁻² U/cm² reconstituted, commercially available, human thrombin (1000 U/ml) to induce polymerization.

The ¹²⁵HBGF-1 was specifically prepared as follows. Fibrinogen was reconstituted by adding 500 mg of fibrinogen into 25 ml of PBS to produce a fibrinogen concentration of 20 mg/ml of PBS. Three ml of this solution which contained 60 mg fibrinogen were placed into 12 Eppendorf plastic tubes and maintained at -70°C. Each of these aliquots was used individually.

The thrombin was reconstituted by diluting a commercially available preparation thereof (Armour Pharmaceutical Co., Kankakee, IL) at a concentration of 1000 U/ml by a factor of 1:10 in sterile solution to produce a concentration of 100 U/ml. This thrombin solution was again diluted 1:10 to produce a solution of 10 U/ml.

The bovine heparin (Upjohn, Kalamazoo, MI) was reconstituted by diluting the preparation at a concentration of 1000 U/ml by a factor of 1:1000 using normal saline.

One and 48/100 (1.48) ml of the reconstituted fibrinogen, plus 63 µL of the reconstituted heparin, plus 15.66 µL of ¹²⁵I-HBGF-1 were mixed in a glass scintillation tube. This mixture was then aspirated into a 3 ml plastic syringe. Five ml of the reconstituted thrombin was placed into a glass scintillation tube.

One end of the expanded PTFE graft was placed over a plastic 3-way stopcock nozzle and was secured there with a 2-0 silk tie. The PTFE was then encircled with a 3 x 3 cm square of Parafilm™ which was then crimped there with a straight hemostat to establish a watertight seal. A second 2-0 silk tie was positioned over the parafilm adjacent to the stopcock to form another seal. A straight hemostat was then used to clamp the distal 2 mm of the PTFE/parafilm to seal this end.

Equal volumes of fibrinogen and thrombin solution prepared as described above were mixed and allowed to react for approximately 30 seconds which is when polymerization occurs. The thrombin-polymerized fibrin is opaque. (This time factor is approximate and varies from one thrombin lot to another. The appropriate length of time to polymerization can be determined by viewing the opacity of the mixture). The fibrin/thrombin mixture was aspirated into a one cc syringe. (NOTE: The volume of this graft was 0.42 ml, for a graft with a larger volume one needs to use a larger syringe.) The syringe was attached to the stopcock and the mixture was injected by hand over a period of 5 seconds until the liquid was seen to "sweat" through the PTFE interstices and filled the space between the PTFE and the Parafilm™. The 3-way stopcock was closed to the PTFE graft for 3 minutes and a scapel blade was used to cut the ligature at the end of the PTFE over the stopcock. The PTFE graft/parafilm was removed from the stopcock and a hemostat was used to remove the PTFE from the parafilm envelope. To clear residual growth factor-supplemented fibrin glue from the graft lumen, a number 3 embolectomy catheter was passed through the graft five times until the graft lumen was completely clear. The growth factor-supplemented fibrin glue-treated PTFE graft was allowed to dry overnight for about 12 hours under a laminar flow hood. The treated graft was then ready for implantation.

This HBGF-supplemented fibrin glue was pressure perfused into a 34 mm (24 mm + 5 mm at each end) x 4 mm (internal diameter) thin-walled, expanded PTFE grafts thereby coating the grafts' luminal surfaces and extending through the nodes to the grafts' outer surfaces. The lumens of the grafts were cleared as stated above. The grafts were then interposed into the infrarenal abdominal aortas of 24, 3-5 Kg New Zealand white rabbits. In the first study, the animals were sacrificed and specimens were explanted at 0 time (to correct for losses due to surgical manipulation) and after 5, 30, and 60 min, and 1, 7, 14, and 30 days. Residual radioactivity was determined by gamma counting. Remaining ¹²⁵I-HBGF-1, corrected for spontaneous decay, is expressed as a percentage of the zero time value.

The washout of ¹²⁵I-HBGF-1 followed classic kinetics with a rapid initial loss with the reestablishment of circulation (%/min = -24.1 between 5 and 60 minutes) followed by a slow loss after 1 hr (%/min = -0.03) with 13.4% ± 6.9% remaining after 1 week and 3.8% ± 1.1% remaining after 30 days.

The second study evaluated the effects of the applied HBGF-1-supplemented fibrin glue suspension on: the rate of endothelialization of widely expanded 60 µm internodal distance expanded PTFE grafts implanted into canine aorto-iliac positions; the proliferative activity of these endothelial cells as a function of time; and the relative contributions of the HBGF-1 and the fibrin glue in stimulating the observed endothelial cell proliferation. Three groups of 50 x 4 mm non-reinforced expanded PTFE grafts were implanted in the aortoiliac position of 12 dogs. Group 1 (n = 6) contained 20 µg/cm² heparin, 2.86 mg/cm² fibrinogen and 2.86 x 10⁻² U/cm² of human thrombin plus 1 ng/cm² of HBGF-1. Group 2 (n = 3) contained the same fibrin glue without HBGF-1. Group 3 (n = 3) consisted of identical but untreated control grafts. Tritiated thymidine (³H-TdR; 0.5 µCi/kg) was injected im 10 hours before explantation. Grafts were explanted at 7 and 28 days for light and electron microscopy, Factor VIII immunohistochemistry, and *en face* autoradiography for endothelial cells proliferation in random high power fields. Each graft was viewed by three "blinded" observers who did not know from which treatment group the graft came. Differences in endothelial cell proliferation were statistically analyzed by two-way ANOVA and independent t-tests.

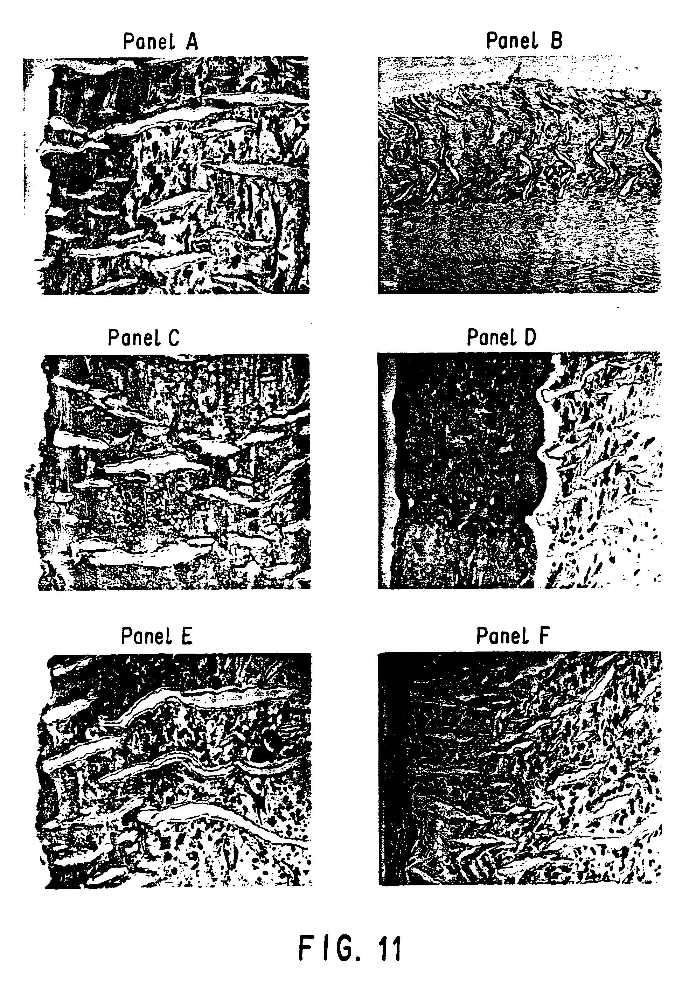

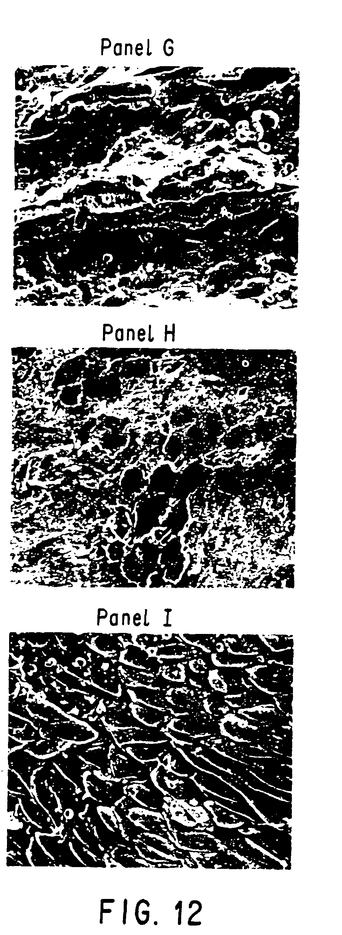

At 7 days 33% of both the fibrin glue and HBGF-1-supplemented fibrin glue demonstrated non-contiguous foci of endothelial cells (Fig. 11). The surface of the control grafts remained a fibrin coagulum. At 28 days, every HBGF-1-supplemented fibrin glue showed extensive capillary ingrowth and confluent endothelialized blood contacting surfaces, which were not seen in any specimen of the other two groups (Figs. 11 and 12). Figure 12 demonstrates that untreated grafts at 28 days had few visible endothelial cells on the surface (Panel G). Grafts treated with fibrin glue alone had about 33% of their surface covered with endothelial cells indicating that fibrin glue treatment alone encouraged some reendothelialization (Panel H). However, grafts treated with fibrin glue supplemented with HBGF-1 (Panel I) appeared to be completely (>95%) covered with endothelial cells which display the characteristic cobblestone morphology. Thus, the combination of growth factors delivered by fibrin glue was able to encourage essentially the complete covering of the vascular graft with a non-thrombogenic endothelial cell lining. *En face* autoradiography revealed a statistically significant increase (p < .05) in ³H-TdR incorporation into the DNA of ECs in the HBGF-1-supplemented fibrin glue grafts at 28 days vs. all other groups both as a function of time and of graft treatment.

These data demonstrate that pressure perfusion of an HBGF-1-supplemented fibrin glue suspension into 60µ internodal distance expanded PTFE grafts promotes endothelialization via capillary ingrowth and increased EC proliferation.

These studies suggest the possibility of enhancing spontaneous reendothelialization of small diameter vascular grafts in man, and also a method for stimulating a more rapid confluence of transplanted endothelial cells.

### EXAMPLE 9

### Preparation of a Platelet-Derived Extract for Use with Fibrin Glue.

Plasma reduced platelets were prepared and pelleted. The supernatant plasma was removed. The pelleted platelets were washed, suspended in buffer containing 50 mM histidine and 0.15 M sodium chloride at pH 6.5, and treated with bovine thrombin. After treatment, the supernatant was collected by centrifugation and aliquots were frozen at -80°C. The extract was thawed and mixed with fibrin glue or other tissue sealants.

### EXAMPLE 10

### Effect of Growth Factor-Supplemented Fibrin Glue on Wound Healing In Vivo.

The effect of growth factor-supplemented fibrin glue on the rate of wound repair in diabetic mice was assessed. Two 6 mm full-thickness skin biopsies on the dorsal part of each of 6 test mice were filled with fibrin glue to which 5 µg of HBGF-1β had been added. Identical biopsies in six mice were left untreated, and in six control mice were filled with unsupplemented fibrin glue. After 9 days, all of the mice were sacrificed and histological preparations of 5 micron thick slices from each of the wounds and surrounding skin were prepared and stained with hematoxylin and eosin.

The extent of wound repair in each sample, which was not identified as to the treatment group from which it came, was "blindly" evaluated by each of three trained analysts, who assessed collagen deposition, reepithelialization, thickness of the granulation tissue and the density of inflammatory cells, fibroblasts and capillaries. Each sample was scored from 1 to 15, ranging from no to complete repair. The samples from the wounds treated with unsupplemented fibrin glue were consistently given the lowest scores and those from the untreated wounds or wounds treated with the growth factor-supplemented fibrin glue were given the highest scores.

### EXAMPLE 11

### Fibrin Glue as a Delivery Vehicle of Osteoinductive Substances

To study the ability of fibrin glue to contain the DBM and allow the migration of cells into the fibrin glue-DBM matrix, pellets made from a mixture of fibrin glue and DBM were implanted intramuscularly (pectoral muscle) and in a calvaria 8 mm diameter defect in Long Evans rats. The fibrin glue-DBM pellets were made using a cylindrical press containing a gap which dimensions were 7 mm diameter and 0.7 m height. Twenty-five mg of DBM obtained from human bones are mixed with 60 ul of FG, and compressed into a disk-shaped pellet. The fibrin glue contained approximately 45 mg of fibrinogen and 15 units of thrombin per ml. Five days after the pellets were implanted, the animals were sacrificed and the pellet with its surrounding muscle and bone tissue was harvested. The samples were cut through the center of the disk and analyzed histologically. The histological analysis of these preparations indicates that the implanted pellets provided a matrix with a predetermined shape. The matrix contained large pore size channels through which the osteoinductive cell penetrated the implant. When the fibrin glue-DBM matrix was made using fibrin glue and human DBM supplemented with BMP's, the body's osteogenic cells penetrated the porous matrix and formed the new bone.

In general, the fibrinogen should be present at a final concentration of 3 to 60 mg/ml, with an optimal concentration of approximately 30 mg/ml. DBM should be present at an approximate concentration of about 50 to 500 mg/ml. The osteoinductive growth factor(s) or BMP's should be present at a concentration of about 1 to 100 ug/ml. The material should be carefully delivered into the gap of the nonunion. New bone should be regenerated in approximately 4 to 6 weeks. The shape of the fibrin glue-DBM matrix has been shown to determine the morphology of the newly formed bone. Therefore, when possible, the fibrin glue-DBM matrix should be made of a predetermined shape. The fibrin glue-DBM matrix in liquid form can be delivered or injected into an irregularly shaped defect where it will polymerize and encourage the bone formation in the filled area.

Since modifications will be apparent to those of skill in the art, it is intended that this invention be limited only by the scope of the appended claims.

## Claims (Claims for the following Contracting State(s): AT, BE, CH, LI, DE, DK, FR, GB, IT, LU, NL, SE)

1. A composition comprising a tissue sealant which is fibrinogen or fibrin glue and a growth factor, wherein the total protein concentration of said sealant is not more than 4.0 mg/ml.

2. A composition as claimed in claim 1, further comprising at least one compound selected from the group consisting of inhibiting compounds and potentiating compounds, wherein said inhibiting compounds inhibit the activities of a component of said sealant that interfere with any of the biological activities of said growth factor, wherein said potentiating compounds potentiate, mediate or enhance the biological activities of said growth factor.

3. A composition as claimed in claim 1 or claim 2, wherein said growth factor is selected from the group consisting of platelet-derived growth factor (PDGF), insulin-binding growth factor-1 (IGF-1), insulin-binding growth factor-2 (IGF-2), epidermal growth factor (EGF), transforming growth factor-α (TGF-α), transforming growth factor β (TGF-β), platelet factor 4 (PF-4), osteogenin and other bone growth factors, collagen growth factors, heparin binding growth factor-1 (HBGF-1), heparin binding growth factor-2 (HGBF-2) and biologically active derivatives of any of said growth factors.

4. A composition as claimed in any one of claims 1 to 3, wherein said growth factor is in the form of a platelet-derived extract.

5. A composition as claimed in any one of claims 2 to 4, wherein said inhibiting or potentiating compound is heparin.

6. The use of a composition as defined in any one of claims 1 to 5 in the preparation of an agent for promoting the healing of wounds.

7. The use as claimed in claim 6, wherein said would is an internal wound.

8. The use as claimed in claim 7, wherein said internal wound is a broken bone.

9. The use as claimed in claim 8, wherein said agent comprises osteogenin.

10. The use as claimed in claim 6, wherein the wound is an external wound.

11. The use of as claimed in claim 10, wherein said external wound is a skin wound.

12. The use as claimed in claim 10, wherein said agent comprises heparin binding growth factor-1.

13. The use of a composition comprising a tissue sealant and a growth factor in the preparation of an agent for promoting the endothelialization of a vascular prosthesis.

14. The use as claimed in claim 13, wherein said vascular prosthesis is composed of polytetrafluoro-ethylene.

15. The use as claimed in claim 14, wherein said polytetrafluoroethylene vascular prosthesis has 60 µm intemodal distance pores.

16. The use as claimed in claim 13, wherein said agent comprises heparin-binding growth factor-1.

17. The use as claimed in claim 13, wherein said composition further comprises at least one compound selected from the group consisting of inhibiting compounds and potentiating compounds, wherein said inhibiting compounds inhibit the activities of a component of said sealant that interfere with any of the biological activities of said growth factor, wherein said potentiating compounds potentiate, mediate or enhance the biological activities of said growth factor.

18. The use as claimed in claim 13, wherein said growth factor is selected from the group consisting of platelet-derived growth factor (PDGF), insulin-binding growth factor-1 (IGF-1), insulin-binding growth factor-2 (IGF-2), epidermal growth factor (EGF), transforming growth factor-α (TGF-α), transforming growth factor-β (TGF- β), platelet factor 4 (PF-4), osteogenin and other bone growth factors, collagen growth factors, heparin binding growth factor-1 (HBGF-1), heparin binding growth factor-2 (HBGF-2) and biologically active derivatives of any of said growth factors.

19. The use as claimed in claim 17, wherein said compound is heparin.

20. The use of a tissue sealant and a growth factor in the preparation of an agent for promoting the *in vitro* proliferation and/or differentiation of animal cells.

21. The use as claimed in claim 20, wherein said cells are endothelial cells.

22. The use as claimed in claim 20, wherein said cells are fibroblasts.

23. The use as claimed in claim 20, wherein said agent comprises fibroblast growth factor-1.

24. A process for promoting the *in vitro* proliferation and/or differentiation of animal cells, comprising placing said cells onto or into a composition comprising a tissue sealant and a growth factor.

25. The process of claim 24, wherein said cells are endothelial cells.

26. The process of claim 24, wherein said cells are fibroblasts.

27. The process as claimed in claim 24, wherein said composition further comprises at least one compound selected from the group consisting of inhibiting compounds and potentiating compounds, herein said inhibiting compounds inhibit the activities of a component of said sealant that interfere with any of the biological activities of said growth factor, wherein said potentiating compounds potentiate, mediate or enhance the biological activities of said growth factor.

28. The process as claimed in claim 24, wherein said growth factor is selected from the group consisting of platelet-derived growth factor (PDGF), insulin-binding growth factor-1 (IGF-1), insulin-binding growth factor-2 (IGF-2), epidermal growth factor (EGF), transforming growth factor-α (TGF-α), transforming growth factor-β (TGF- β), platelet factor 4 (PF-4), osteogenin and other bone growth factors, collagen growth factors, heparin binding growth factor-1 (HBGF-1), heparin binding growth factor-2 (HBGF-2) and biologically active derivatives of any said growth factors.

29. The process as claimed in claim 27, wherein said compound is heparin.

30. An *in vitro* process for coating the surface of a biomaterial, comprising applying a composition to said biomaterial, said composition comprising a tissue sealant and a growth factor.

31. The process of claim 30, wherein said biomaterial is a vascular prosthesis and wherein the process promotes the endothelialization of the vascular prosthesis.

32. The *in vitro* process as claimed in claim 30, wherein said composition further comprises at least one compound selected from the group consisting of inhibiting compounds and potentiating compounds, wherein said inhibiting compounds inhibit the activities of a component of said sealant that interfere with any of the biological activities of said growth factor, wherein said potentiating compounds potentiate, mediate or enhance the biological activities of said growth factor.

33. The *in vitro* process as claimed in claim 30, wherein said growth factor is selected from the group consisting of platelet-derived growth factor (PDGF), insulin-binding growth factor-1 (IGF-1), insulin-binding growth factor-2 (IGF-2), epidermal growth factor (EGF), transforming growth factor-α (TGF-α), transforming growth factor- β (TGF- β), platelet factor (PF-4), osteogenin and other bone growth factors, collagen growth factors, heparin binding growth factor-1 (HBGF-1), heparin binding growth factor-2 (HBGF-2) and biologically active derivatives of any of said growth factors.

34. The *in vitro* process as claimed in claim 32, wherein said compound is heparin.

## Claims (Claims for the following Contracting State(s): ES, GR)

1. A process for the preparation of a composition comprising a tissue sealant which is fibrinogen or fibrin glue, wherein the total protein concentration of said sealant is not more than 4.0 mg/ml; the process comprising admixing the components.

2. A process for the preparation of a composition as defined in claim 1 which further comprises at least one compound selected from the group consisting of inhibiting compounds and potentiating compounds, wherein said inhibiting compounds inhibit the activities a component of said sealant that interfere with any of the biological activities of said growth factor, wherein said potentiating compounds potentiate, mediate or enhance the biological activities of said growth factor.

3. A process as claimed in claim 1 or claim 2, wherein said growth factor is selected from the group consisting of platelet-derived growth factor (PDGF), insulin-binding growth factor-1 (IGF-1), insulin-binding growth factor-2 (IGF-2), epidermal growth factor (EGF), transforming growth factor-α (TGF-α), transforming growth factor-β (TGF-β), platelet factor 4 (PF-4), osteogenin and other bone growth factors, collagen growth factors, heparin binding growth factor-1 (HBGF-1), heparin binding growth factor-2 (HBGF-2) and biologically active derivatives of any of said growth factors.

4. A process as claimed in any one of claims 1 to 3, wherein said growth factor is in the form of a platelet-derived extract.

5. A process as claimed in any one of claims 2 to 4, wherein said inhibiting or potentiating compound is heparin.

6. The use of composition as defined in any one of claims 1 to 5 in the preparation of an agent for promoting the healing of wounds.

7. The use as claimed in claim 6, wherein said wound is an internal wound.

8. The use as claimed in claim 7, wherein said internal wound is a broken bone.

9. The use as claimed in claim 8, wherein said agent comprises osteogenin.

10. The use as claimed in claim 6, wherein the wound is an extemal wound.

11. The use as claimed in claim 10, wherein said external wound is a skin wound.

12. The use as claimed in claim 10, wherein said agent comprises heparin binding growth factor-1.

13. The use of a composition comprising a tissue sealant and a growth factor in the preparation of an agent for promoting the endothelialization of a vascular prosthesis.

14. The use as claimed in claim 13, wherein said vascular prosthesis is composed of polytetrafluoro-ethylene.

15. The use as claimed in claim 14, wherein said polytetrafluoroethylene vascular prosthesis has 60 µm intemodal distance pores.

16. The use as claimed in claim 13, wherein said agent comprises heparin-binding growth factor-1.

17. The use as claimed in claim 13, wherein said composition further comprises at least one compound selected from the group consisting of inhibiting compounds and potentiating compounds, wherein said inhibiting compounds inhibit the activities of a component of said sealant that interfere with any of the biological activities of said growth factor, wherein said potentiating compounds potentiate, mediate or enhance the biological activities of said growth factor.

18. The use as claimed in claim 13, wherein said growth factor is selected from the group consisting of platelet-derived growth factor (PDGF), insulin-binding growth factor-1 (IGF-1), insulin-binding growth factor-2 (IGF-2), epidermal growth factor (EGF), transforming growth factor-α (TGF-α), transforming growth factor-β (TGF- β), platelet factor 4 (PF-4), osteogenin and other bone growth factors, collagen growth factors, heparin binding growth factor-1 (HBGF-1), heparin binding growth factor-2 (HBGF-2) and biologically active derivatives of any of said growth factors.

19. The use as claimed in claim 17, wherein said compound is heparin.

20. The use of a tissue sealant and a growth factor in the preparation of an agent for promoting the *in vitro* proliferation and/or differentiation of animal cells.

21. The use as claimed in claim 20, wherein said cells are endothelial cells.

22. The use as claimed in claim 20, wherein said cells are fibroblasts.

23. The use as claimed in claim 20, wherein said agent comprises fibroblast growth factor-1.

24. A process for promoting the *in vitro* proliferation and/or differentiation of animal cells, comprising placing said cells onto or into a composition comprising a tissue sealant and a growth factor.

25. The process of claim 24, wherein said cells are endothelial cells.

26. The process of claim 24, wherein said cells are fibroblasts.

27. The process as claimed in claim 24, wherein said composition further comprises at least one compound selected from the group consisting of inhibiting compounds and potentiating compounds, herein said inhibiting compounds inhibit the activities of a component of said sealant that interfere with any of the biological activities of said growth factor, wherein said potentiating compounds potentiate, mediate or enhance the biological activities of said growth factor.

28. The process as claimed in claim 24, wherein said growth factor is selected from the group consisting of platelet-derived growth factor (PDGF), insulin-binding growth factor-1 (IGF-1), insulin-binding growth factor-2 (IGF-2), epidermal growth factor (EGF), transforming growth factor-α (TGF-α), transforming growth factor-β (TGF-β), platelet factor 4 (PF-4), osteogenin and other bone growth factors, collagen growth factors, heparin binding growth factor-1 (HBGF-1), heparin binding growth factor-2 (HBGF-2) and biologically active derivatives of any said growth factors.

29. The process as claimed in claim 27, wherein said compound is heparin.

30. An *in vitro* process for coating the surface of a biomaterial, comprising applying a composition to said biomaterial, said composition comprising a tissue sealant and a growth factor.

31. The process of claim 30, wherein said biomaterial is a vascular prosthesis and wherein the process promoted the endothelialization of the vascular prosthesis.

32. The in *vitro* process as claimed in claim 30, wherein said composition further comprises at least one compound selected from the group consisting of inhibiting compounds and potentiating compounds, wherein said inhibiting compounds inhibit the activities of a component of said sealant that interfere with any of the biological activities of said growth factor, wherein said potentiating compounds potentiate, mediate or enhance the biological activities of said growth factor.

33. The *in vitro* process as claimed in claim 30, wherein said growth factor is selected from the group consisting of platelet-derived growth factor (PDGF), insulin-binding growth factor-1 (IGF-1), insulin-binding growth factor-2 (IGF-2), epidermal growth factor (EGF), transforming growth factor-α (TGF-α), transforming growth factor-β (TGF- β), platelet factor (PF-4), osteogenin and other bone growth factors, collagen growth factors, heparin binding growth factor-1 (HBGF-1), heparin binding growth factor-2 (HBGF-2) and biologically active derivatives of any of said growth factors.

34. The *in vitro* process as claimed in claim 32, wherein said compound is heparin.

## Patentansprüche (Patentansprüche für folgende(n) Vertragsstaat(en): AT, BE, CH, LI, DE, DK, FR, GB, IT, LU, NL, SE)

1. Zusammensetzung, enthaltend ein Gewebeverschlussmittel, das Fibrinogen oder Fibrin-Kleber ist, und einen Wachstumsfaktor, bei der die Gesamt-Proteinkonzentration des Verschlussmittels nicht mehr als 4,0 mg/ml beträgt.

2. Zusammensetzung gemäß Anspruch 1, die weiterhin mindestens eine Verbindung enthält, die aus der Gruppe ausgewählt ist, die aus hemmenden Verbindungen und verstärkenden Verbindungen besteht, wobei die hemmenden Verbindungen die Aktivitäten einer Verbindung des Verschlussmittels hemmen, die mit irgendeiner der biologischen Aktivitäten des Wachstumsfaktors interferieren, wobei die verstärkenden Verbindungen die biologischen Aktivitäten des Wachstumsfaktors verstärken, weitergeben oder verbessern.

3. Zusammensetzung gemäß Anspruch 1 oder 2, bei der der Wachstumsfaktor aus der Gruppe ausgewählt ist, die aus Plättchenwachstumsfaktor (PDGF), Insulinbindungswachstumsfaktor-1 (IGF-1), Insulinbindungswachstumsfaktor-2 (IGF-2), epidermalem Wachstumsfaktor (EGF), Transformationswachstumsfaktor-α (TGF-α), Transformationswachstumsfaktor-β (TGF-β), Plättchenfaktor 4 (PF-4), Osteogenin und anderen Knochenwachstumsfaktoren, Kollagenwachstumsfaktoren, Heparinbindungswachstumsfaktor-1 (HBGF-1), Heparinbindungswachstumsfaktor-2 (HBGF-2) und biologisch aktiven Derivaten irgendeiner dieser Wachstumsfaktoren besteht.

4. Zusammensetzung gemäß irgendeinem der Ansprüche 1 bis 3, bei der der Wachstumsfaktor in der Form eines Plättchenextraktes vorliegt.

5. Zusammensetzung gemäß irgendeinem der Ansprüche 2 bis 4, bei der die hemmende oder verstärkende Verbindung Heparin ist.

6. Verwendung einer Zusammensetzung gemäß irgendeinem der Ansprüche 1 bis 5 zur Herstellung eines Mittels zur Förderung der Wundheilung.

7. Verwendung gemäß Anspruch 6, bei der die Wunde eine innere Wunde ist.

8. Verwendung gemäß Anspruch 7, bei der die innere Wunde ein gebrochener Knochen ist.

9. Verwendung gemäß Anspruch 8, bei der das Mittel Osteogenin enthält.

10. Verwendung gemäß Anspruch 6, bei der die Wunde eine äußere Wunde ist.

11. Verwendung gemäß Anspruch 10, bei der die äußere Wunde eine Hautwunde ist.

12. Verwendung gemäß Anspruch 10, bei der das Mittel Heparinbindungswachstumsfaktor-1 enthält.

13. Verwendung einer Zusammensetzung, die ein Gewebeverschlussmittel und einen Wachstumsfaktor zur Herstellung eines Mittels zur Förderung der Endothelialisierung einer Gefäßprothese enthält.

14. Verwendung gemäß Anspruch 13, bei der die GeAßprothese aus Polytetrafluorethylen gebildet ist.

15. Verwendung gemäß Anspruch 14, bei der die Polytetrafluorethylen-Gefäßprothese 60 µm internodale Distanz-Poren aufweisen.

16. Verwendung gemäß Anspruch 13, bei der das Mittel Heparinbindungswachstumsfaktor-1 enthält.

17. Verwendung gemäß Anspruch 13, bei der die Zusammensetzung weiterhin mindestens eine Verbindung enthält, die aus der Gruppe ausgewählt ist, die aus hemmenden Verbindungen und verstärkenden Verbindungen besteht, wobei die hemmenden Verbindungen die Aktivitäten einer Verbindung des Verschlussmittels hemmen, die mit irgendeiner der biologischen Aktivitäten des Wachstumsfaktors interferieren, wobei die verstärkenden Verbindungen die biologischen Aktivitäten des Wachstumsfaktors verstärken, weitergeben oder verbessern.

18. Verwendung gemäß Anspruch 13, bei der der Wachstumsfaktor aus der Gruppe ausgewählt wird, die aus Plättchenwachstumsfaktor (PDGF), Insulinbindungswachstumsfaktor-1 (IGF-1), Insulinbindungswachstumsfaktor-2 (IGF-2), epidermalem Wachstumsfaktor (EGF), Transformationswachstumsfaktor-α (TGF-α), Transformationswachstumsfaktor-β (TGF-β), Plättchenfaktor 4 (PF-4), Osteogenin und anderen Knochenwachstumsfaktoren, Kollagenwachstumsfaktoren, Heparinbindungswachstumsfaktor-1 (HBGF-1), Heparinbindungswachstumsfaktor-2 (HBGF-2) und biologisch aktiven Derivaten irgendeiner dieser Wachstumsfaktoren besteht.

19. Verwendung gemäß Anspruch 17, bei der die Verbindung Heparin ist.

20. Verwendung eines Gewebeverschlussmittels und eines Wachstumsfaktors zur Herstellung eines Mittels zur Förderung der *in vitro* Proliferation und/oder Differenzierung von tierischen Zellen.

21. Verwendung gemäß Anspruch 20, bei der die Zellen endotheliale Zellen sind.

22. Verwendung gemäß Anspruch 20, bei der die Zellen Fibroblasten sind.

23. Verwendung gemäß Anspruch 20, bei der das Mittel Fibroblastwachstumsfaktor-1 enthält.

24. Verfahren zur Förderung der *in vitro* Proliferation und/oder Differenzierung von tierischen Zellen, das plazieren dieser Zellen auf oder in eine Zusammensetzung umfasst, die ein Gewebeverschlussmittel oder einen Wachstumsfaktor enthält.

25. Verfahren gemäß Anspruch 24, bei dem die Zellen endotheliale Zellen sind.

26. Verfahren gemäß Anspruch 24, bei dem die Zellen Fibroblasten sind.

27. Verfahren gemäß Anspruch 24, bei dem die Zusammensetzung weiterhin mindestens eine Verbindung enthält, die aus der Gruppe ausgewählt wird, die aus hemmenden Verbindungen und verstärkenden Verbindungen besteht, wobei die hemmenden Verbindungen die Aktivitäten einer Verbindung des Verschlussmittels hemmen, die mit irgendeiner der biologischen Aktivitäten des Wachstumsfaktors interferieren, wobei die verstärkenden Verbindungen die biologischen Aktivitäten des Wachstumsfaktors verstärken, weitergeben oder verbessern.

28. Verfahren gemäß Anspruch 24, bei dem der Wachstumsfaktor aus der Gruppe ausgewählt wird, die aus Plättchenwachstumsfaktor (PDGF), Insulinbindungswachstumsfaktor-1 (IGF-1), Insulinbindungswachstumsfaktor-2 (IGF-2), epidermalem Wachstumsfaktor (EGF), Transformationswachstumsfaktor-α (TGF-α), Transformationswachstumsfaktor-β (TGF-β), Plättchenfaktor 4 (PF-4), Osteogenin und anderen Knochenwachstumsfaktoren, Kollagenwachstumsfaktoren, Heparinbindungswachstumsfaktor-1 (HBGF-1), Heparinbindungswachstumsfaktor-2 (HBGF-2) und biologisch aktiven Derivaten irgendeiner dieser Wachstumsfaktoren besteht.

29. Verfahren gemäß Anspruch 27, bei der die Verbindung Heparin ist.

30. *In vitro* Verfahren zur Beschichtung der Oberfläche eines Biomaterials, das aufbringen einer Zusammensetzung auf das Biomaterial enthält, wobei die Zusammensetzung ein Gewebeverschlussmittel und einen Wachstumsfaktor enthält.

31. Verfahren gemäß Anspruch 30, bei dem das Biomaterial eine Gefäßprothese ist und wobei das Verfahren die Endothelialisierung der Gefäßprothese fördert.

32. *In vitro* Verfahren gemäß Anspruch 30, bei dem die Zusammensetzung weiterhin mindestens eine Verbindung enthält, die aus der Gruppe ausgewählt wird, die aus hemmenden Verbindungen und verstärkenden Verbindungen besteht, wobei die hemmenden Verbindungen die Aktivitäten einer Verbindung des Verschlussmittels hemmen, die mit irgendeiner der biologischen Aktivitäten des Wachstumsfaktors interferieren, wobei die verstärkenden Verbindungen die biologischen Aktivitäten des Wachstumsfaktors verstärken, weitergeben oder verbessem.

33. *In vitro* Verfahren gemäß Anspruch 30, bei dem der Wachstumsfaktor aus der Gruppe ausgewählt wird, die aus Plättchenwachstumsfaktor (PDGF), Insulinbindungswachstumsfaktor-1 (IGF-1), Insulinbindungswachstumsfaktor-2 (IGF-2), epidermalem Wachstumsfaktor (EGF), Transformationswachstumsfaktor-α (TGF-α), Transformationswachstumsfaktor-β (TGF-β), Plättchenfaktor 4 (PF-4), Osteogenin und anderen Knochenwachstumsfaktoren, Kollagenwachstumsfaktoren, Heparinbindungswachstumsfaktor-1 (HBGF-1), Heparinbindungswachstumsfaktor-2 (HBGF-2) und biologisch aktiven Derivaten irgendeiner dieser Wachstumsfaktoren besteht.

34. *In vitro* Verfahren gemäß Anspruch 32, bei dem die Verbindung Heparin ist.

## Patentansprüche (Patentansprüche für folgende(n) Vertragsstaat(en): ES, GR)

1. Verfahren zur Herstellung einer Zusammensetzung, die ein Gewebeverschlussmittel enthält, das Fibrinogen oder Fibrin-Kleber ist, in der die Gesamt-Proteinkonzentration des Verschlussmittels nicht mehr als 4,0 mg/ml beträgt; wobei das Verfahren Mischen der Verbindungen enthält.

2. Verfahren zur Herstellung einer Zusammensetzung gemäß Anspruch 1, die weiterhin mindestens eine Verbindung enthält, die aus der Gruppe ausgewählt wird, die aus hemmenden Verbindungen und verstärkenden Verbindungen besteht, wobei die hemmenden Verbindungen die Aktivitäten einer Verbindung des Verschlussmittels hemmen, die mit irgendeiner der biologischen Aktivitäten des Wachstumsfaktors interferieren, wobei die verstärkenden Verbindungen die biologischen Aktivitäten des Wachstumsfaktors verstärken, weitergeben oder verbessern.

3. Verfahren gemäß Anspruch 1 oder 2, in dem der Wachstumsfaktor aus der Gruppe ausgewählt wird, die aus Plättchenwachstumsfaktor (PDGF), Insulinbindungswachstumsfaktor-1 (IGF-1), Insulinbindungswachstumsfaktor-2 (IGF-2), epidermalem Wachstumsfaktor (EGF), Transformationswachstumsfaktor-α (TGF-α), Transformationswachstumsfaktor-β (TGF-β), Plättchenfaktor 4 (PF-4), Osteogenin und anderen Knochenwachstumsfaktoren, Kollagenwachstumsfaktoren, Heparinbindungswachstumsfaktor-1 (HBGF-1), Heparinbindungswachstumsfaktor-2 (HBGF-2) und biologisch aktiven Derivaten irgendeiner dieser Wachstumsfaktoren besteht.

4. Verfahren gemäß irgendeinem der Ansprüche 1 bis 3, in dem der Wachstumsfaktor in der Form eines Plättchenextraktes vorliegt.

5. Verfahren gemäß irgendeinem der Ansprüche 2 bis 4, in dem die hemmende oder verstärkende Verbindung Heparin ist.

6. Verwendung einer Zusammensetzung gemäß irgendeinem der Ansprüche 1 bis 5 zur Herstellung eines Mittels zur Förderung der Wundheilung.

7. Verwendung gemäß Anspruch 6, bei der die Wunde eine innere Wunde ist.

8. Verwendung gemäß Anspruch 7, bei der die innere Wunde ein gebrochener Knochen ist.

9. Verwendung gemäß Anspruch 8, bei der das Mittel Osteogenin enthält.

10. Verwendung gemäß Anspruch 6, bei der die Wunde eine äußere Wunde ist.

11. Verwendung gemäß Anspruch 10, bei der die äußere Wunde eine Hautwunde ist.

12. Verwendung gemäß Anspruch 10, bei der das Mittel Heparinbindungswachstumsfaktor-1 enthält.

13. Verwendung einer Zusammensetzung, die ein Gewebeverschlussmittel und einen Wachstumsfaktor zur Herstellung eines Mittels zur Förderung der Endothelialisierung einer Gefäßprothese enthält.

14. Verwendung gemäß Anspruch 13, bei der die Gefäßprothese aus Polytetrafluorethylen gebildet ist.

15. Verwendung gemäß Anspruch 14, bei der die Polytetrafluorethylen-Gefäßprothese 60 µm internodale Distanz-Poren aufweisen.

16. Verwendung gemäß Anspruch 13, bei der das Mittel Heparinbindungswachstumsfaktor-1 enthält.

17. Verwendung gemäß Anspruch 13, bei der die Zusammensetzung weiterhin mindestens eine Verbindung enthält, die aus der Gruppe ausgewählt wird, die aus hemmenden Verbindungen und verstärkenden Verbindungen besteht, wobei die hemmenden Verbindungen die Aktivitäten einer Verbindung des Verschlussmittels hemmen, die mit irgendeiner der biologischen Aktivitäten des Wachstumsfaktors interferieren, wobei die verstärkenden Verbindungen die biologischen Aktivitäten des Wachstumsfaktors verstärken, weitergeben oder verbessern.

18. Verwendung gemäß Anspruch 13, bei der der Wachstumsfaktor aus der Gruppe ausgewählt wird, die aus Plättchenwachstumsfaktor (PDGF), Insulinbindungswachstumsfaktor-1 (IGF-1), Insulinbindungswachstumsfaktor-2 (IGF-2), epidermalem Wachstumsfaktor (EGF), Transformationswachstumsfaktor-α (TGF-α), Transformationswachstumsfaktor-β (TGF-β), Plättchenfaktor 4 (PF-4), Osteogenin und anderen Knochenwachstumsfaktoren, Kollagenwachstumsfaktoren, Heparinbindungswachstumsfaktor-1 (HBGF-1), Heparinbindungswachstumsfaktor-2 (HBGF-2) und biologisch aktiven Derivaten irgendeiner dieser Wachstumsfaktoren besteht.

19. Verwendung gemäß Anspruch 17, bei der die Verbindung Heparin ist.

20. Verwendung eines Gewebeverschlussmittels und eines Wachstumsfaktors zur Herstellung eines Mittels zur Förderung der *in vitro* Proliferation und/oder Differenzierung von tierischen Zellen.

21. Verwendung gemäß Anspruch 20, bei der die Zellen endotheliale Zellen sind.

22. Verwendung gemäß Anspruch 20, bei der die Zellen Fibroblasten sind.

23. Verwendung gemäß Anspruch 20, bei der das Mittel Fibroblastwachstumsfaktor-1 enthält.

24. Verfahren zur Förderung der *in vitro* Proliferation und/oder Differenzierung von tierischen Zellen, das plazieren dieser Zellen auf oder in eine Zusammensetzung enthält, die ein Gewebeverschlussmittel oder einen Wachstumsfaktor enthält.

25. Verfahren gemäß Anspruch 24, bei dem die Zellen endotheliale Zellen sind.

26. Verfahren gemäß Anspruch 24, bei dem die Zellen Fibroblasten sind.

27. Verfahren gemäß Anspruch 24, bei dem die Zusammensetzung weiterhin mindestens eine Verbindung enthält, die aus der Gruppe ausgewählt wird, die aus hemmenden Verbindungen und verstärkenden Verbindungen besteht, wobei die hemmenden Verbindungen die Aktivitäten einer Verbindung des Verschlussmittels hemmen, die mit irgendeiner der biologischen Aktivitäten des Wachstumsfaktors interferieren, wobei die verstärkenden Verbindungen die biologischen Aktivitäten des Wachstumsfaktors verstärken, weitergeben oder verbessern.

28. Verfahren gemäß Anspruch 24, bei dem der Wachstumsfaktor aus der Gruppe ausgewählt ist, die aus Plättchenwachstumsfaktor (PDGF), Insulinbindungswachstumsfaktor-1 (IGF-1), Insulinbindungswachstumsfaktor-2 (IGF-2), epidermalem Wachstumsfaktor (EGF), Transformationswachstumsfaktor-α (TGF-α), Transformationswachstumsfaktor-β (TGF-β), Plättchenfaktor 4 (PF-4), Osteogenin und anderen Knochenwachstumsfaktoren, Kollagenwachstumsfaktoren, Heparinbindungswachstumsfaktor-1 (HBGF-1), Heparinbindungswachstumsfaktor-2 (HBGF-2) und biologisch aktive Derivate irgendeiner dieser Wachstumsfaktoren besteht.

29. Verfahren gemäß Anspruch 27, bei der die Verbindung Heparin ist.

30. *In vitro* Verfahren zur Beschichtung der Oberfläche eines Biomaterials, das aufbringen einer Zusammensetzung auf das Biomaterial umfasst, wobei die Zusammensetzung ein Gewebeverschlussmittel und einen Wachstumsfaktor enthält.

31. Verfahren gemäß Anspruch 30, bei dem das Biomaterial eine Gefäßprothese ist und wobei das Verfahren die Endothelialisierung der Gefäßprothese fördert.

32. *In vitro* Verfahren gemäß Anspruch 30, bei dem die Zusammensetzung weiterhin mindestens eine Verbindung enthält, die aus der Gruppe ausgewählt wird, die aus hemmenden Verbindungen und verstärkenden Verbindungen besteht, wobei die hemmenden Verbindungen die Aktivitäten einer Verbindung des Verschlussmittels hemmen, die mit irgendeiner der biologischen Aktivitäten des Wachstumsfaktors interferieren, wobei die verstärkenden Verbindungen die biologischen Aktivitäten des Wachstumsfaktors verstärken, weitergeben oder verbessern.

33. *In vitro* Verfahren gemäß Anspruch 30, bei dem der Wachstumsfaktor aus der Gruppe ausgewählt wird, die aus Plättchenwachstumsfaktor (PDGF), Insulinbindungswachstumsfaktor-1 (IGF-1), Insulinbindungswachstumsfaktor-2 (IGF-2), epidermalem Wachstumsfaktor (EGF), Transformationswachstumsfaktor-α (TGF-α), Transformationswachstumsfaktor-β (TGF-β), Plättchenfaktor 4 (PF-4), Osteogenin und anderen Knochenwachstumsfaktoren, Kollagenwachstumsfaktoren, Heparinbindungswachstumsfaktor-1 (HBGF-1), Heparinbindungswachstumsfaktor-2 (HBGF-2) und biologisch aktiven Derivaten irgendeiner dieser Wachstumsfaktoren besteht.

34. *In vitro* Verfahren gemäß Anspruch 32, bei dem die Verbindung Heparin ist.

## Revendications (Revendications pour l'(les) Etat(s) contractant(s) suivant(s): AT, BE, CH, LI, DE, DK, FR, GB, IT, LU, NL, SE)

1. Composition comprenant un colmatant tissulaire qui est le fibrinogêne ou une colle de fibrine et un facteur de croissance, où la concentration en protéines totales dudit colmatant ne dépasse pas 4,0 mg/ml.

2. Composition selon la revendication 1, comprenant en outre au moins un composé choisi dans l'ensemble constitué par des composés inhibiteurs et des composés potentiateurs, où lesdits composés inhibiteurs inhibent les activités d'un composant dudit colmatant qui interfèrent avec l'une quelconque des activités biologiques dudit facteur de croissance, lesdits composés potentiateurs ayant un effet potentialisant, médiateur ou amplificateur des activités biologiques dudit facteur de croissance.

3. Composition selon la revendication 1 ou la revendication 2, dans laquelle ledit facteur de croissance est choisi dans le groupe constitué par le facteur de croissance dérivé des plaquettes (PDGF), le facteur 1 de croissance de liaison à l'insuline (IGF-1), le facteur 2 de croissance de liaison à l'insuline (IGF-2), le facteur de croissance de l'epiderme (EGF), le facteur α de transformation (TGF-α), le facteur β de transformation (TGF-β), le facteur plaquettaire (PF-4), l'ostéogénine et d'autres facteurs de croissance des os, les facteurs de croissance du collagène, le facteur 1 de croissance de liaison à l'héparine (HBGF-1), le facteur 2 de croissance de liaison à l'héparine (HGBF-2), et les dérivés biologiquement actifs de l'un quelconque desdits facteurs de croissance.

4. Composition selon l'une quelconque des revendications 1 à 3, dans laquelle ledit facteur de croissance est sous la forme d'un extrait issu de plaquettes.

5. Composition selon l'une quelconque des revendications 2 à 4, dans laquelle ledit composé inhibiteur ou potentiateur est l'héparine.

6. Utilisation d'une composition telle que définie dans l'une quelconque des revendications 1 à 5, dans la préparation d'un agent pour favoriser la cicatrisation de plaies.

7. Utilisation selon la revendication 6, dans laquelle ladite plaie est une plaie interne.

8. Utilisation selon la revendication 7, dans laquelle ladite plaie interne est un os fracturé.

9. Utilisation selon la revendication 8, dans laquelle ledit agent comprend l'ostéogénine.

10. Utilisation selon la revendication 6, dans laquelle la plaie est une plaie externe.

11. Utilisation selon la revendication 10, dans laquelle ladite plaie externe est une plaie cutanée.

12. Utilisation selon la revendication 10, dans laquelle ledit agent comprend le facteur 1 de croissance de liaison à l'héparine.

13. Utilisation d'une composition comprenant un colmatant tissulaire et un facteur de croissance, dans la préparation d'un agent pour favoriser l'endothélialisation d'une prothèse vasculaire.

14. Utilisation selon la revendication 13, dans laquelle ladite prothèse vasculaire est composée de polytétrafluoroéthylène.

15. Utilisation selon la revendication 14, dans laquelle ladite prothèse vasculaire en polytétrafluoroéthylène a des pores ayant une distance internodale de 60 µm.

16. Utilisation selon la revendication 13, dans laquelle ledit agent comprend le facteur 1 de croissance de liaison à l'héparine.

17. Utilisation selon la revendication 13, dans laquelle ladite composition comprend en outre au moins un composé choisi dans l'ensemble constitué par des composés inhibiteurs et des composés potentiateurs, où lesdits composés inhibiteurs inhibent les activités d'un composant dudit colmatant qui interfèrent avec l'une quelconque des activités biologiques dudit facteur de croissance, lesdits composés potentiateurs ayant un effet potentialisant, médiateur ou amplificateur des activités biologiques dudit facteur de croissance.

18. Utilisation selon la revendication 13, dans laquelle ledit facteur de croissance est choisi dans le groupe constitué par le facteur de croissance issu des plaquettes (PDGF), le facteur 1 de croissance de liaison à l'insuline (IGF-1), le facteur 2 de croissance de liaison à l'insuline (IGF-2), le facteur de croissance de l'épiderme (EGF), le facteur α de transformation (TGF-α), le facteur β de transformation (TGF-β), le facteur plaquettaire 4 (PF-4), l'ostéogénine et d'autres facteurs de croissance des os, les facteurs de croissance du collagène, le facteur 1 de croissance de liaison à l'héparine (HBGF-1), le facteur 2 de croissance de liaison à l'héparine (HGBF-2), et les dérivés biologiquement actifs de l'un quelconque desdits facteurs de croissance.

19. Utilisation selon la revendication 17, dans laquelle ledit composé est l'héparine.

20. Utilisation d'un colmatant tissulaire et d'un facteur de croissance dans la préparation d'un agent pour favoriser la prolifération et/ou la différenciation in vitro de cellules animales.

21. Utilisation selon la revendication 20, dans laquelle lesdites cellules sont des cellules endothéliales.

22. Utilisation selon la revendication 20, dans laquelle lesdites cellules sont des fibroblastes.

23. Utilisation selon la revendication 20, dans laquelle ledit agent comprend le facteur 1 de croissance des fibroblastes.

24. Procédé pour favoriser la prolifération et/ou la différenciation in vitro de cellules animales comprenant la mise en place desdites cellules sur ou dans une composition comprenant un colmatant tissulaire et un facteur de croissance.

25. Procédé selon la revendication 24, dans lequel lesdites cellules sont des cellules endothéliales.

26. Procédé selon la revendication 24, dans lequel lesdites cellules sont des fibroblastes.

27. Procédé selon la revendication 24, dans lequel ladite composition comprend en outre au moins un composé choisi dans l'ensemble constitué par des composés inhibiteurs et des composés potentiateurs, où lesdits composés inhibiteurs inhibent les activités d'un composant dudit colmatant qui interfèrent avec l'une quelconque des activités biologiques dudit facteur de croissance, lesdits composés potentiateurs ayant un effet potentialisant, médiateur ou amplificateur des activités biologiques dudit facteur de croissance.

28. Procédé selon la revendication 24, dans lequel ledit facteur de croissance est choisi dans le groupe constitué par le facteur de croissance issu des plaquettes (PDGF), le facteur 1 de croissance de liaison à l'insuline (IGF-1), le facteur 2 de croissance de liaison à l'insuline (IGF-2), le facteur de croissance de l'épiderme (EGF), le facteur α de transformation (TGF-α), le facteur β de transformation (TGF-β), le facteur plaquettaire (PF-4), l'ostéogénine et d'autres facteurs de croissance des os, les facteurs de croissance du collagène, le facteur 1 de croissance de liaison à l'héparine (HBGF-1), le facteur 2 de croissance de liaison à l'héparine (HGBF-2), et les dérivés biologiquement actifs de l'un quelconque desdits facteurs de croissance.

29. Procédé selon la revendication 27, dans lequel ledit composé est l'héparine.

30. Procédé in vitro pour revêtir la surface d'un biomatériau, comprenant l'application d'une composition sur le biomatériau, ladite composition comprenant un colmatant tissulaire et un facteur de croissance.

31. Procédé selon la revendication 30, dans lequel ledit biomatériau est une prothèse vasculaire, lequel procédé favorise l'endothélialisation de la prothèse vasculaire.

32. Procédé in vitro selon la revendication 30, dans lequel ladite composition comprend en outre au moins un composé choisi dans l'ensemble constitué par des composés inhibiteurs et des composés potentiateurs, où lesdits composés inhibiteurs inhibent les activités d'un composant dudit colmatant qui interfèrent avec l'une quelconque des activités biologiques dudit facteur de croissance, lesdits composés potentiateurs ayant un effet potentialisant, médiateur ou amplificateur des activités biologiques dudit facteur de croissance.

33. Procédé in vitro selon la revendication 30, dans lequel ledit facteur de croissance est choisi dans le groupe constitué par le facteur de croissance issu des plaquettes (PDGF), le facteur 1 de croissance de liaison à l'insuline (IGF-1), le facteur 2 de croissance de liaison à l'insuline (IGF-2), le facteur de croissance de l'épiderme (EGF), le facteur α de transformation (TGF-α), le facteur β de transformation (TGF-β), le facteur plaquettaire (PF-4), l'ostéogénine et d'autres facteurs de croissance des os, les facteurs de croissance du collagêne, le facteur 1 de croissance de liaison à l'héparine (HBGF-1), le facteur 2 de croissance de liaison à l'héparine (HGBF-2), et les dérivés biologiquement actifs de l'un quelconque desdits facteurs de croissance.

34. Procédé in vitro selon la revendication 32, dans lequel ledit composé est l'héparine.

## Revendications (Revendications pour l'(les) Etat(s) contractant(s) suivant(s): ES, GR)

1. Procédé pour la préparation d'une composition comprenant un colmatant tissulaire qui est le fibrinogène ou une colle de fibrine et un facteur de croissance, où la concentration en protéines totales dudit colmatant ne dépasse pas 4,0 mg/ml ; le procédé comprenant le mélange des composants.

2. Procédé pour la préparation d'une composition tel que défini dans la revendication 1, laquelle composition comprend en outre au moins un composé choisi dans l'ensemble constitué par des composés inhibiteurs et des composés potentiateurs, où lesdits composés inhibiteurs inhibent les activités d'un composant dudit colmatant qui interfèrent avec l'une quelconque des activités biologiques dudit facteur de croissance, lesdits composés potentiateurs ayant un effet potentialisant, médiateur ou amplificateur des activités biologiques dudit facteur de croissance.

3. Procédé selon la revendication 1 ou la revendication 2, dans lequel ledit facteur de croissance est choisi dans le groupe constitué par le facteur de croissance dérivé des plaquettes (PDGF), le facteur 1 de croissance de liaison à l'insuline (IGF-1), le facteur 2 de croissance de liaison à l'insuline (IGF-2), le facteur de croissance de l'épiderme (EGF), le facteur α de transformation (TGF-α), le facteur β de transformation (TGF-β), le facteur plaquettaire 4 (PF-4), l'ostéogénine et d'autres facteurs de croissance des os, les facteurs de croissance du collagène, le facteur 1 de croissance de liaison à l'héparine (HBGF-1), le facteur 2 de croissance de liaison à l'héparine (HGBF-2), et les dérivés biologiquement actifs de l'un quelconque desdits facteurs de croissance.

4. Procédé selon l'une quelconque des revendications 1 à 3, dans lequel ledit facteur de croissance est sous la forme d'un extrait dérivant de plaquettes.

5. Procédé selon l'une quelconque des revendications 2 à 4, dans lequel ledit composé inhibiteur ou potentiateur est l'héparine.

6. Utilisation d'une composition telle que définie dans l'une quelconque des revendications 1 à 5, dans la préparation d'un agent pour favoriser la cicatrisation de plaies.

7. Utilisation selon la revendication 6, dans laquelle ladite plaie est une plaie interne.

8. Utilisation selon la revendication 7, dans laquelle ladite plaie interne est un os fracturé.

9. Utilisation selon la revendication 8, dans laquelle ledit agent comprend de l'ostéogénine.

10. Utilisation selon la revendication 6, dans laquelle la plaie est une plaie externe.

11. Utilisation selon la revendication 10, dans laquelle ladite plaie externe est une plaie cutanée.

12. Utilisation selon la revendication 10, dans laquelle ledit agent comprend le facteur 1 de croissance de liaison à l'héparine.

13. Utilisation d'une composition comprenant un colmatant tissulaire et un facteur de croissance, dans la préparation d'un agent pour favoriser l'endothélialisation d'une prothèse vasculaire.

14. Utilisation selon la revendication 13, dans laquelle ladite prothèse vasculaire est composée de polytétrafluoroéthylène.

15. Utilisation selon la revendication 14, dans laquelle ladite prothèse vasculaire en polytétrafluoroéthylène a des pores ayant une distance internodale de 60 µm.

16. Utilisation selon la revendication 13, dans laquelle ledit agent comprend le facteur 1 de croissance de liaison à l'héparine.

17. Utilisation selon la revendication 13, dans laquelle ladite composition comprend en outre au moins un composé choisi dans l'ensemble constitué par des composés inhibiteurs et des composés potentiateurs, où lesdits composés inhibiteurs inhibent les activités d'un composant dudit colmatant qui interfèrent avec l'une quelconque des activités biologiques dudit facteur de croissance, lesdits composés potentiateurs ayant un effet potentialisant, médiateur ou amplificateur des activités biologiques dudit facteur de croissance.

18. Utilisation selon la revendication 13, dans laquelle ledit facteur de croissance est choisi dans le groupe constitué par le facteur de croissance dérivé des plaquettes (PDGF), le facteur 1 de croissance de liaison à l'insuline (IGF-1), le facteur 2 de croissance de liaison à l'insuline (IGF-2), le facteur de croissance de l'épiderme (EGF), le facteur α de transformation (TGF-α), le facteur β de transformation (TGF-β), le facteur plaquettaire (PF-4), l'ostéogénine et d'autres facteurs de croissance des os, les facteurs de croissance du collagène, le facteur 1 de croissance de liaison à l'héparine (HBGF-1), le facteur 2 de croissance de liaison à l'héparine (HGBF-2), et les dérivés biologiquement actifs de l'un quelconque desdits facteurs de croissance.

19. Utilisation selon la revendication 17, dans laquelle ledit composé est l'héparine.

20. Utilisation d'un colmatant tissulaire et d'un facteur de croissance dans la préparation d'un agent pour favoriser la prolifération et/ou la différenciation in vitro de cellules animales.

21. Utilisation selon la revendication 20, dans laquelle lesdites cellules sont des cellules endothéliales.

22. Utilisation selon la revendication 20, dans laquelle lesdites cellules sont des fibroblastes.

23. Utilisation selon la revendication 20, dans laquelle ledit agent comprend le facteur 1 de croissance des fibroblastes.

24. Procédé pour favoriser la prolifération et/ou la différenciation in vitro de cellules animales comprenant la mise en place desdites cellules sur ou dans une composition comprenant un colmatant tissulaire et un facteur de croissance.

25. Procédé selon la revendication 24, dans lequel lesdites cellules sont des cellules endothéliales.

26. Procédé selon la revendication 24, dans lequel lesdites cellules sont des fibroblastes.

27. Procédé selon la revendication 24, dans lequel ladite composition comprend en outre au moins un composé choisi dans l'ensemble constitué par des composés inhibiteurs et des composés potentiateurs, où lesdits composés inhibiteurs inhibent les activités d'un composant dudit colmatant qui interfèrent avec l'une quelconque des activités biologiques dudit facteur de croissance, lesdits composés potentiateurs ayant un effet potentialisant, médiateur ou amplificateur des activités biologiques dudit facteur de croissance.

28. Procédé selon la revendication 24, dans lequel ledit facteur de croissance est choisi dans le groupe constitué par le facteur de croissance dérivé des plaquettes (PDGF), le facteur 1 de croissance de liaison à l'insuline (IGF-1), le facteur 2 de croissance de liaison à l'insuline (IGF-2), le facteur de croissance de l'épiderme (EGF), le facteur α de transformation (TGF-α), le facteur β de transformation (TGF-β), le facteur plaquettaire (PF-4), l'ostéogénine et d'autres facteurs de croissance des os, les facteurs de croissance du collagène, le facteur 1 de croissance de liaison à l'héparine (HBGF-1), le facteur 2 de croissance de liaison à l'héparine (HGBF-2), et les dérivés biologiquement actifs de l'un quelconque desdits facteurs de croissance.

29. Procédé selon la revendication 27, dans lequel ledit composé est l'héparine.

30. Procédé in vitro pour revêtir la surface d'un biomatériau, comprenant l'application d'une composition sur ledit biomatériau, ladite composition comprenant un colmatant tissulaire et un facteur de croissance.

31. Procédé selon la revendication 30, dans lequel ledit biomatériau est une prothèse vasculaire, lequel procédé favorise l'endothélialisation de la prothèse vasculaire.

32. Procédé in vitro selon la revendication 30, dans lequel ladite composition comprend en outre au moins un composé choisi dans l'ensemble constitué par des composés inhibiteurs et des composés potentiateurs, où lesdits composés inhibiteurs inhibent les activités d'un composant dudit colmatant qui interfèrent avec l'une quelconque des activités biologiques dudit facteur de croissance, lesdits composés potentiateurs ayant un effet potentialisant, médiateur ou amplificateur des activités biologiques dudit facteur de croissance.

33. Procédé in vitro selon la revendication 30, dans lequel ledit facteur de croissance est choisi dans le groupe constitué par le facteur de croissance dérivé des plaquettes (PDGF), le facteur 1 de croissance de liaison à l'insuline (IGF-1), le facteur 2 de croissance de liaison à l'insuline (IGF-2), le facteur de croissance de l'épiderme (EGF), le facteur α de transformation (TGF-α), le facteur β de transformation (TGF-β), le facteur plaquettaire 4 (PF-4), l'ostéogénine et d'autres facteurs de croissance des os, les facteurs de croissance du collagène, le facteur 1 de croissance de liaison à l'héparine (HBGF-1), le facteur 2 de croissance de liaison à l'héparine (HGBF-2), et les dérivés biologiquement actifs de l'un quelconque desdits facteurs de croissance.

34. Procédé in vitro selon la revendication 32, dans lequel ledit composé est l'héparine.
